# EUROPEAN PATENT SPECIFICATION

(11) **EP 2 188 280 B1**
(45) Date of publication and mention of the grant of the patent: **09.03.2011**
(21) Application number: 08788941.6
(22) Date of filing: 22.07.2008
(51) Int. Cl.: C07D 405/14, C07D 471/04, A61P 31/12, A61K 31/437

(54) **IMIDAZOPYRIDINONES**
IMIDAZOPYRIDINONE
IMIDAZOPYRIDINONES

(30) Priority: 03.08.2007 US 953707 P
(43) Date of publication of application: 26.05.2010
(73) Proprietor: Pfizer Limited, Sandwich, Kent CT13 9NJ (GB)
(72) Inventor: JONES, Peter, Sandwich, Kent CT13 9NJ (GB); PRYDE, David, Cameron, Sandwich, Kent CT13 9NJ (GB); TRAN, Thien, Duc, Sandwich, Kent CT13 9NJ (GB)
(74) Representative: Kingsbury, Oliver William
(86) International application number: PCT/IB2008/001962
(87) International publication number: WO 2009/019553

(56) References cited:
- WO-A-2007/028129
- WO-A-2007/093901

## Description

The invention relates to imidazopyridinones, to their use in medicine, to compositions containing them, to processes for their preparation and to intermediates used in such processes.

Toll-Like Receptors (TLRs) are primary transmembrane proteins characterized by an extracellular leucine-rich domain and a cytoplasmic tail that contains a conserved region named the Toll/IL-1 receptor (TIR) domain. They are expressed predominantly on immune cells (for example dendritic cells, T lymphocytes, macrophages, monocytes and natural killer cells), which serve as a key part of the innate immune system. They are a group of pattern recognition receptors which bind to pathogen-associated molecular patterns (for reviews see, for example, Ulevitch, R. J., Nature Reviews: Immunology, 4, 512-520, 2004; and Akira, S., Takeda, K., and Kaisho, T., Annual Rev. Immunol., 21, 335-376, 2003). Their name derives from sequence homology to the Drosophila melanogaster gene Toll, which was found in fruit flies to play a key role in protecting the fly from fungal infections (Hoffmann, J. A., Nature, 426, 33-38, 2003). Eleven TLRs have been identified in mammalian systems; non-mammalian TLRs have been found in other vertebrates. All TLRs appear to function as either a homodimer or heterodimer in the recognition of a specific, or set of specific, molecular determinants present on pathogenic organisms, including bacterial cell-surface lipopolysaccharides, lipoproteins, bacterial flagellin, DNA from both bacteria and viruses and viral RNA. The cellular response to TLR activation involves activation of one or more transcription factors, leading to the production and secretion of cytokines and co-stimulatory molecules such as interferons, TNF-α, interleukins, MIP-1 and MCP-1, which contribute to the killing and clearance of the pathogenic invasion. By activating TLRs, it should be possible to induce or stimulate immune cells to mount an immune response. In particular, TLR7 has been implicated in a number of disorders (see, for example, Kanzler et al, Nature Medicine 2007, Vol 13, No 5, pp 552-559), including viral infections (such as HCV or HBV), cancers and tumours, and T2 Helper cell (TH2) mediated diseases, and hence TLR7 agonists are potentially useful in the treatment of such diseases. TLRs can also play a critical role in regulation of both innate and adaptive immunity (see, for example, Parker et al, Clinical and Experimental Immunology 2007, 199-207.

Certain imidazopyridinones said to induce interferon-α and hence to treat viral diseases, are known from WO2007/028129.

There is, however, an ongoing need to provide new TLR7 agonists that are good drug candidates. In particular, such compounds should bind potently to TLR7, whilst showing little affinity for other receptors, and show functional activity as TLR7 agonists. They should be well absorbed from the gastrointestinal tract, be metabolically stable and possess favourable pharmacokinetic properties (such as swift onset of action and minimal 'food effect'). They should be non-toxic and demonstrate few side-effects. Furthermore, the ideal drug candidate will have good aqueous solubility and exist in a physical form that is stable, non-hygroscopic and easily formulated.

We have now found a series of imidazopyridinones which are agonists of TLR7.

According to a first aspect of the invention, therefore, there is provided a compound of formula (I) 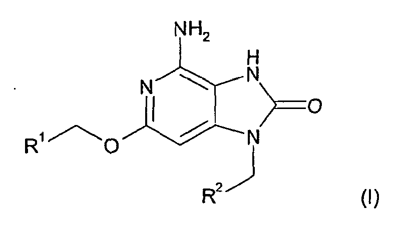 or a pharmaceutically acceptable salt or solvate thereof, wherein
R¹ is 3- to 8-membered saturated heterocyclic group wherein one ring member is -O-; and
R² is phenyl or pyridinyl, each optionally substituted by C₁-C₆alkyl.

Unless otherwise indicated, alkyl groups may be straight or branched and contain 1 to 6 carbon atoms and preferably 1 to 4 carbon atoms. Examples of alkyl include methyl, ethyl, n-propyl, isopropyl, n-butyl, isobutyl, sec-butyl, pentyl and hexyl.

In one embodiment of the invention, R¹ is tetrahydropyranyl or tetrahydrofuranyl. In another embodiment, R¹ is tetrahydropyranyl. In another embodiment, R¹ is tetrahydropyran-4-yl.

In another embodiment of the invention, R² is pyridinyl, optionally substituted by C₁-C₄alkyl. In another embodiment of the invention, R² is pyridinyl, optionally substituted by methyl. In the forgoing embodiments, R² is preferably pyridin-3-yl, i.e.:

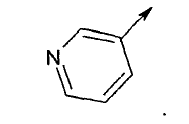

In another embodiment, R² is 6-methyl-pyridin-3-yl, i.e.:

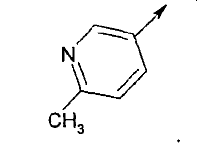

In another embodiment of the invention, there is provided a compound selected from:
4-Amino-1-(6-methyl-pyridin-3-ylmethyl)-6-(tetrahydro-pyran-4-ylmethoxy)-1,3-dihydro-imidazo[4,5-c]pyridin-2-one;
4-Amino-1-(6-methyl-pyridin-3-ylmethyl)-6-(tetrahydro-furan-3-R/S-ylmethoxy)-1,3-dihydro-imidazo[4,5-c]pyridin-2-one;
4-Amino-1-(6-methyl-pyridin-3-ylmethyl)-6-(tetrahydro-furan-3-S-ylmethoxy)-1,3-dihydro-imidazo[4,5-c]pyridin-2-one;
4-Amino-1-(6-methyl-pyridin-3-ylmethyl)-6-(tetrahydro-furan-3-R-ylmethoxy)-1,3-dihydro-imidazo[4,5-c]pyridin-2-one;
4-Amino-1-(6-methyl-pyridin-3-ylmethyl)-6-(tetrahydro-furan-2-R/S-ylmethoxy)-1,3-dihydro-imidazo[4,5-c]pyridin-2-one;
4-Amino-1-(6-methyl-pyridin-3-ylmethyl)-6-(tetrahydro-furan-2-R-ylmethoxy)-1,3-dihydro-imidazo[4,5-c]pyridin-2-one;
4-Amino-1-(6-methyl-pyridin-3-ylmethyl)-6-(tetrahydro-furan-2-S-ylmethoxy)-1,3-dihydro-imidazo[4,5-c]pyridin-2-one;
or a pharmaceutically acceptable salt or solvate thereof.

4-Amino-1-(6-methyl-pyridin-3-ylmethyl)-6-(tetrahydro-pyran-4-ylmethoxy)-1,3-dihydro-imidazo[4,5-c]pyridin-2-one, or a pharmaceutically acceptable salt or solvate thereof, is a preferred compound of the invention.

Pharmaceutically acceptable salts of the compounds of formula (I) comprise the acid addition and base salts thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate; tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

The skilled person will appreciate that the aforementioned salts include ones wherein the counterion is optically active, for example d-lactate or I-lysine, or racemic; for example dl-tartrate or dl-arginine.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (I) may be prepared by one or more of three methods:
(i) by reacting the compound of formula (I) with the desired acid or base;
(ii) by removing an acid- or base-labile protecting group from a suitable precursor of the compound of formula (I) using the desired acid or base; or
(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the ' resulting salt may vary from completely ionised to almost non-ionised.

The compounds of formula (I) or pharmaceutically acceptable salts thereof may exist in both unsolvated and solvated forms. The term 'solvate' is used herein to describe a molecular complex comprising the compound of formula (I) or a pharmaceutically acceptable salt thereof and one or more pharmaceutically acceptable solvent molecules, for example, ethanol. The term 'hydrate' is employed when said solvent is water. Pharmaceutically acceptable solvates in accordance with the invention include those wherein the solvent of crystallization may be isotopically substituted, e.g. D₂O, d₆-acetone and d₆-DMSO.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion. When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order (melting point').

Also included within the scope of the invention are multi-component complexes (other than salts and solvates) of compounds of formula (I) or pharmaceutically acceptable salts thereof wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through noncovalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004). For a general review of multi-component'complexes, see J Pharm Sci, 64 (8), 1269-1288, by Haleblian (August 1975).

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K⁺, or -SO₃⁻Na⁺) or non-ionic (such as -N⁻N⁺(CH₃)₃) polar head group. For more information, see Crystals and the Polarizing Microscope by N. H. Hartshorne and A. Stuart, 4^{th} Edition (Edward Arnold, 1970).

Hereinafter, all references to compounds of the invention include compounds of formula (I) or pharmaceutically acceptable salt, solvates, or multi-component complexes thereof, or pharmaceutically acceptable solvates or multi-component complexes of pharmaceutically acceptable salts of compounds of formula (I).

Preferred compounds of the invention are compounds of formula (I) or pharmaceutically acceptable salts or solvates thereof.

Certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as 'prodrugs'. [Further information on the use of prodrugs may be found in 'Pro-drugs as Novel Delivery Systems, Vol. 14, ACS Symposium Series (T Higuchi and W Stella) and 'Bioreversible Carriers in Drug Design', Pergamon Press, 1987 (ed. E B Roche, American Pharmaceutical Association).]

Prodrugs can, for example, be produced by replacing appropriate functionalities present in the compounds of formula (I) with certain moieties known to those skilled in the art as 'pro-moieties' as described, for example, in "Design of Prodrugs" by H Bundgaard (Elsevier, 1985).

Examples of prodrugs include amides of the compounds of formula (I), wherein, as the case may be, one or both hydrogens of the 4-amino functionality of the compounds of formula (I) is/are replaced by (C₁-C₁₀)alkanoyl.

Further examples of replacement groups in accordance with the foregoing examples and examples of other prodrug types may be found in the aforementioned references.

Also described are metabolites of compounds of formula (I), that is, compounds formed *in vivo* upon administration of the drug. Some examples of metabolites include
(i) where the compound of formula (I) contains a methyl group, an hydroxymethyl derivative thereof (-CH₃ -> -CH₂OH):
(ii) where the compound of formula (I) contains a phenyl (Ph) moiety, a phenol derivative thereof (-Ph > -PhOH);
(iii) where the compound of formula (I) contains a pyridinyl (Pyr) moiety, a hydroxypyridinyl derivative thereof (-Pyr > -PyrOH);

Compounds of the invention containing one or more asymmetric carbon atoms (e.g. where R¹ is tetrahydrofuranyl) can exist as two or more stereoisomers. Where structural isomers are interconvertible via a low energy barrier, tautomeric isomerism ('tautomerism') can occur. This can take the form of proton tautomerism in compounds of the invention containing, for example, a keto group, or so-called valence tautomerism in compounds which contain an aromatic moiety. It follows that a single compound may exhibit more than one type of isomerism.

In particular, compounds of the invention exhibit keto-enol tautomerism, as follows:

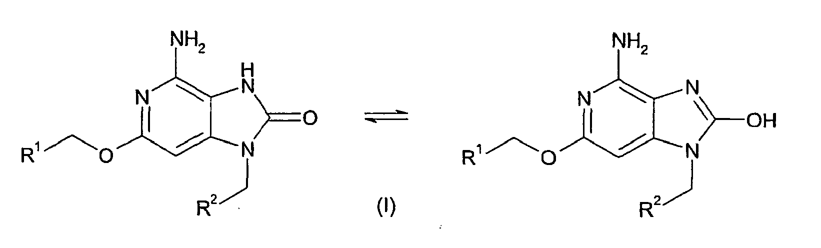

Included within the scope of the invention are all stereoisomers and tautomeric forms of the compounds of the invention, including compounds exhibiting more than one type of isomerism, and mixtures of one or more thereof.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1 % diethylamine. Concentration of the eluate affords the enriched mixture.

Mixtures of stereoisomers may be separated by conventional techniques known to those skilled in the art; see, for example, "Stereochemistry of Organic Compounds" by E. L. Eliel and S. H. Wilen (Wiley, New York, 1994.

The scope of the invention includes all crystal forms of the compounds of the compounds of the invention, including racemates and racemic mixtures (conglomerates) thereof. Stereoisomeric conglomerates may also be separated by the conventional techniques described herein just above.

Also described are pharmaceutically acceptable isotopically-labelled compounds of the invention wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I , nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (I), for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagent in place of the non-labeled reagent previously employed.

When preparing compounds of formula (I) in accordance with the invention, it is open to a person skilled in the art to routinely select the form of intermediate which provides the best combination of features for this purpose. Such features include the melting point, solubility, processability and yield of the intermediate form and the resulting ease with which the product may be purified on isolation.

The compounds of the invention may be prepared by any method known in the art for the preparation of compounds of analogous structure. In particular, the compounds of the invention can be prepared by the procedures described by reference to Scheme 1 that follows, or by the specific methods described in the Examples, or by similar processes to either.

It will be further appreciated that it may be necessary or desirable to carry out the transformations in a different order from that described in the schemes, or to modify one or more of the transformations, to provide the desired compound of the invention.

In addition, the skilled person will appreciate that it may be necessary or desirable at any stage in the synthesis of compounds of the invention to protect one or more sensitive groups, so as to prevent undesirable side reactions. In particular, it may be necessary or desirable to protect amino groups. The protecting groups used in the preparation of compounds of the compounds of the invention may be used in conventional manner. See, for example, those described in 'Greene's Protective Groups in Organic Synthesis' by Theodora W Greene and Peter G M Wuts, fourth edition, (John Wiley and Sons, 2006), in particular chapter 7 ("Protection for the Amino Group"), which also describes methods for the removal of such groups. The amino protecting groups *t*-butoxycarbonyl, benzyloxycarbonyl, benzyl and acetyl are of particular use in the preparation of compounds of formula (I) and intermediates thereto.

Unless otherwise indicated, R¹ and R² in Scheme 1 that follows are as defined herein. Each LG¹, which may be the same or different, is a leaving group appropriate to facilitate aromatic nucleophilic substitution by the source of ammonia in step (e). Such groups include halogen, such as chloro, and sulphonic esters, such as tosylate, mesylate or triflate. LG² is a leaving group appropriate to facilitate aliphatic nucleophilic substitution by the amino group in step (d). Such groups also include halogen, such as bromo, and sulphonic esters, such as tosylate, mesylate or triflate. PG¹ is an alkoxycarbonyl (e.g. ethoxycarbonyl) amino protecting group, which serves both to protect the amino group during earlier synthetic steps of Scheme 1 and, in step h, reacts with an amino group in the cyclisation to provide the desired imidazopyridinones of formula (I); the skilled person would be well able to select other suitable protecting groups, as for example described in the aforementioned 'Greene's Protective Groups in Organic Synthesis'. 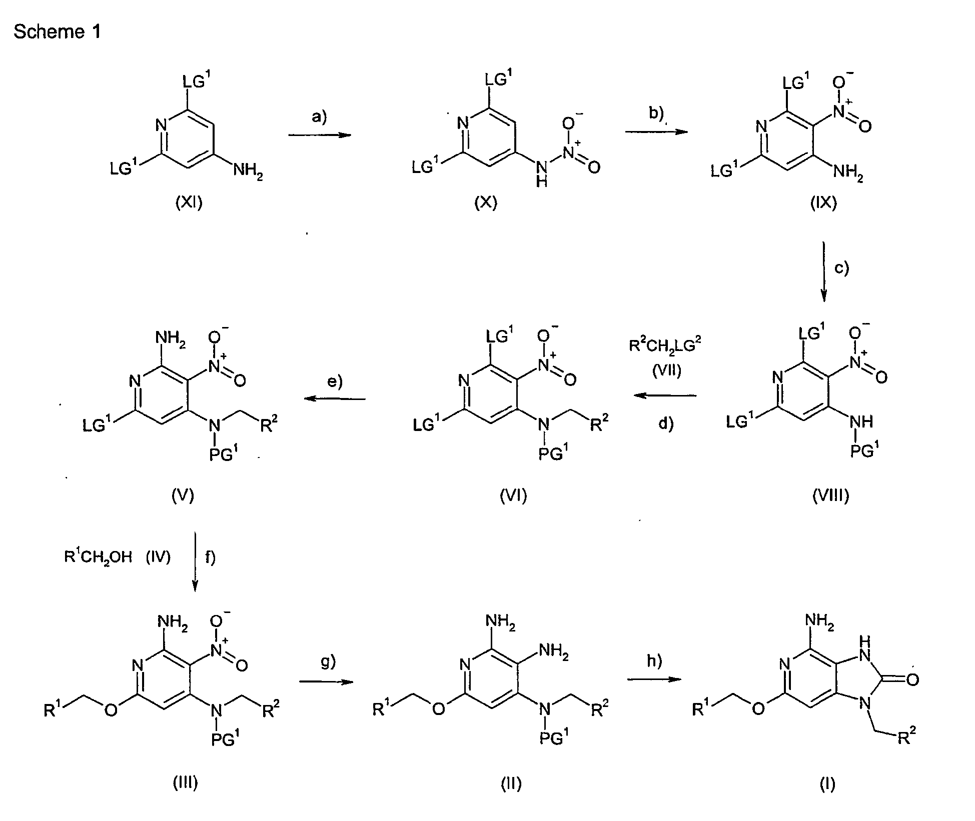
a) Amines of formula (XI) may be treated under conventional nitration conditions known to those skilled in the art to provide the corresponding (N-nitro)amine of formula (X). Convenient nitration conditions are described in Preparation 1 that follows.
b) (N-nitro)amines of formula (X) are rearranged to the corresponding isomeric nitro compound of formula (IX) under conventional conditions known to those skilled in the art. Conveniently, the rearrangement is effected under acid conditions, for example see the analogous chemistry described in the procedures on pp 41-42 of W02005026164.
c) The amine group in amines of formula (IX) may be protected under conventional conditions known to those skilled in the art to give the corresponding protected derivative of formula (VIII). Conveniently the amino group is protected in the form of an alkyl carbamate by treating the amine of formula (IX) with a strong base, such as an alkali metal base (e.g. sodium hydride, potassium tert-butoxide or lithium diisopropylamide); in the presence of an acylating agent, such as alkyl or aryl chloroformate (e.g. ethylchloroformate or cyanoformate), or an acid anhydride, such as alkyl - or aryl acid anhydride, (e.g. acetic anhydride).
d) Compounds of formula (VIII) may be alkylated with a compound of formula (VII), such as a benzyl halide (e.g. benzyl bromide) or a halomethylpyridine (e.g. 5-(chloromethyl)-2-methylpyridine), under conventional conditions known to those skilled in the art, to give the corresponding compound of formula (VI). Conveniently, the alkylation is effected using a mild base, such as an alkali metal carbonate (e.g. potassium carbonate or sodium carbonate); in the presence of an iodide source, such as an alkali metal iodide (e.g. sodium iodide); and in an organic solvent, such as a polar aprotic solvent (e.g. acetone).
e) Compounds of formula (VI) may be treated with a source of ammonia, such as ammonia or an ammonium salt (e.g. ammonium acetate), under conventional conditions known to those skilled in the art to give the corresponding amino pyridine of formula (V). Convenient reaction conditions are described in Preparations 6 and 7 that follow.
f) The LG¹ in amino pyridines of formula (V) may be displaced through reaction with an alcohol of formula (IV), under conventional conditions known to those skilled in the art, to give the corresponding ether of formula (III). Conveniently, the displacement is effected in the presence of a strong base, such as an alkali metal base (e.g. sodium hydride, potassium tert-butoxide or sodium hexamethyldisilazide); and in an organic solvent, such as an ether (e.g. tetrahydrofuran).
g) The nitro group in compounds of formula (III) may be reduced to give the corresponding amine of formula (II) under conventional conditions known to those skilled in the art. Conveniently, the reduction may be effected by a metal, such as iron or tin, and in the presence of an acid, such as an inorganic acid (e.g. HCl). In one alternative, the reduction may be effected by hydrogenation in the presence of a transition metal catalyst, such as palladium, platinum or nickel, and in a solvent, such as an alcohol (e.g. ethanol). In another alternative, the reduction may be effected by a metal hydride, such as an alkali metal hydride (e.g. lithium aluminium hydride), or sodium dithionate, and in a solvent, such as an ether (e.g. diethyl ether); and at reduced temperature, such as from -78°C to 0°C.
h) The amines of formula (II) can be cyclised to the corresponding imidazopyridinones of formula (I) by treatment with a protic acid, under conventional conditions. Conveniently, the protic acid is an organic acid, such as acetic acid or formic acid, or an inorganic acid, such as hydrochloric acid, and the reaction is performed at ambient to elevated temperature, such as elevated temperature (e.g. in the region 40 - 80°C).

In another aspect there is described a process for preparing a compound of formula (I) which comprises treating a compound of formulae (II), or a salt or solvate thereof, with a protic acid under conventional cyclisation conditions.

Compounds of formulae (XI), (VII) and (IV) are either commercially available, known from the literature or easily prepared by methods well known to those skilled in the art.

Compounds of the invention intended for pharmaceutical use may be administered as crystalline or amorphous products or may exist in a continuum of solid states ranging from fully amorphous to fully crystalline. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

In another aspect the invention provides a pharmaceutical composition comprising a compound of the invention together with one or more pharmaceutically acceptable excipients.

Pharmaceutical compositions suitable for the delivery of compounds of the present invention and methods for their preparation will be readily apparent to those skilled in the art. Such compositions and methods for their preparation may be found, for example, in "Remington's Pharmaceutical Sciences", 19th Edition (Mack Publishing Company, 1995).

Suitable modes of administration include oral, parenteral, topical, inhaled/intranasal, rectal/intravaginal, and ocular/aural administration.

Formulations suitable for the aforementioned modes of administration may be formulated to be immediate and/or modified release. Modified release formulations include delayed-, sustained-, pulsed-, controlled-, targeted and programmed release.

The compounds of the invention may be administered orally. Oral administration may involve swallowing, so that the compound enters the gastrointestinal tract, or buccal or sublingual administration may be employed by which the compound enters the blood stream directly from the mouth. Formulations suitable for oral administration include solid formulations such as tablets, capsules containing particulates, liquids, or powders, lozenges (including liquid-filled), chews, multi- and nano-particulates, gels, solid solution, liposome, films, ovules, sprays, liquid formulations and buccal/mucoadhesive patches.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet. Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. The formulation of tablets is discussed in "Pharmaceutical Dosage Forms: Tablets", Vol. 1, by H. Lieberman and L. Lachman (Marcel Dekker, New York, 1980).

Suitable modified release formulations for the purposes of the invention are described in US Patent No. 6,106,864. Details of other suitable release technologies such as high energy dispersions and osmotic and coated particles are to be found in "Pharmaceutical Technology On-line", 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release. Thus compounds of the invention may be formulated as a solid, semisolid, or thixotropic liquid for administration as an implanted depot providing modified release of the active compound. Examples of such formulations include drug-coated stents and poly(dl-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject™, Bioject™, etc.) injection.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (I), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

In the case of dry powder inhalers and aerosols, the dosage unit is determined by means of a valve which delivers a metered amount. Units in accordance with the invention are typically arranged to administer a metered dose or "puff' containing from 1µg to 100mg of the compound of formula (I). The overall daily dose will typically be in the range 1µg to 200mg which may be administered in a single dose or, more usually, as divided doses throughout the day.

The compounds of the invention may be administered rectally or vaginally, for example, in the form of a suppository, pessary, microbicide, vaginal ring or enema. Cocoa butter is a traditional suppository base, but various alternatives may be used as appropriate.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

For administration to human patients, the total daily dose of the compounds of the invention is typically in the range 1 mg to 10g, such as 10mg to 1g, for example 25mg to 500mg depending, of course, on the mode of administration and efficacy. For example, oral administration may require a total daily dose of from 50mg to 100mg. The total daily dose may be administered in single or divided doses and may, at the physician's discretion, fall outside of the typical range given herein. These dosages are based on an average human subject having a weight of about 60kg to 70kg. The physician will readily be able to determine doses for subjects whose weight falls outside this range, such as infants and the elderly.

As noted above, by agonising TLR7 it should be possible to induce or stimulate immune cells to mount an immune response in response to, for example, viral infections (such as HCV or HBV), cancers and tumours, and T2 Helper cell (TH2) mediated diseases. Thus the compounds of the invention are useful because they exhibit pharmacological activity, i.e. TLR7 agonism, in animals. More particularly, the compounds of the invention are of use in the treatment of disorders for which a TLR7 agonist is indicated. Preferably the animal is a mammal, more preferably a human.

In a further aspect of the invention there is provided a compound of the invention for use as a medicament.

In a further aspect of the invention there is provided a compound of the invention for the treatment of a disorder for which a TLR7 agonist is indicated.

In a further aspect of the invention there is provided use of a compound of the invention for the preparation of a medicament for the treatment of a disorder for which a TLR7 agonist is indicated.

Disorders for which a TLR7 agonist is indicated include viral infections, such as infections caused by adenovirus, herpesvirus (e.g. HSV-I, HSV-II, CMV, or VZV), poxvirus (e.g. orthopoxvirus such as variola or vaccinia, or molluscum contagiosum), picornavirus (e.g. rhinovirus or enterovirus), orthomyxovirus (e.g. influenzavirus), paramyxovirus (e.g. parainfluenzavirus, mumps virus, measles virus, or respiratory syncytial virus (RSV)), coronavirus (e.g. SARS), papovavirus (e.g. papillomaviruses, such as those that cause genital warts, common warts, or plantar warts), hepadnavirus (e.g., hepatitis B virus), flavivirus (e.g. hepatitis C virus or Dengue virus), retrovirus (e.g. a lentivirus such as HIV) or filovirus (e.g. ebola virus or marbug virus); bacterial infections, such as infections caused by bacteria of the genus Escherichia, Enterobacter, Salmonella, Staphylococcus, Klebsiella, Proteus, Pseudomonas, Streptococcus, Chlamydia; fungal infections, such as candidiasis, aspergillosis, histoplasmosis, cryptococcal meningitis; and, and parasitic infections, such as protozoal infections (e.g.malaria).

Use of the compounds of the invention in the treatment of viral infections, such as hepatitis C virus (HCV) infections, is of particular interest.

Further disorders for which a TLR7 agonist is indicated include tumours or cancers, including carcinomas, sarcomas and leukemias, such as squamous cell carcinoma, renal cell carcinoma, Kaposi's sarcoma, melanoma, renal cell carcinoma, myelogeous leukemia, chronic lymphocytic leukemia, multiple myeloma, non- Hodgkin's lymphoma.

Still further disorders for which a TLR7 agonist is indicated include T- helper cells (Th2) mediated diseases (see e.g. Dabbagh et al., Curr Opin Infect Dis 2003, 16: 199-204), including but not limited to atopic diseases, such as atopic dermatitis or eczema, eosinophilia, asthma, allergy, allergic rhinitis.

Still further disorders for which a TLR7 agonist is indicated include damaged or ageing skin such as scarring and wrinkles.

Still further disorders for which a TLR7 agonist is indicated include autoimmune diseases, such as Crohns disease and inflammatory bowel disease.

The compounds of the invention may also be used as vaccine adjuvants, in particular in the treatment of viral infections and tumours or cancers. Use of the compounds of the invention as vaccine adjuvants is of particular interest in the treatment of HCV and HIV infections.

The compounds of the invention may be administered alone or as part of a combination therapy. Thus co-administration of a compound of the invention and one or more additional therapeutic agents is included within the scope of the present invention. Such combinations offer the possibility of significant advantages, including patient compliance, ease of dosing and synergistic activity.

In a further aspect of the invention there is provided a pharmaceutical composition including one or more additional therapeutic agents.

In one embodiment, combinations of the present invention include a compound of the invention and one or more additional agents useful in the treatment of HCV. Such additional agents include HCV fusion inhibitors, such as E1 antagonists or E2 antagonists; HCV NS2 inhibitors; HCV NS3 inhibitors (e.g. VX-950, SCH-503034, ITMN-191); HCV NS4A inhibitors; HCV NS4B inhibitors; HCV NS5A inhibitors (e.g. A-831); HCV NS5B inhibitors (e.g. PSI-6130, valopicitabine, HCV-796, R-1479, GS-9190 or 6-cyclopentyl-6-(2-(2,6-diethylpyridin-4-yl)ethyl)-3-((5,7-dimethyl-[1,2,4]triazolo[1,5-a]pyrimidin-2-yl)methyl)-4-hydroxy-5,6-dihydropyran-2-one); HCV metalloprotease inhibitors; HCV helicase inhibitors; HCV p7 inhibitors; inosine monophosphate dehydrogenase (IMPDH) inhibitors (e.g. viramidine, meremepodib); interferons, such as pegylated interferons (e.g. peginterferon alfa-2a and peginterferon alfa-2b) or long-acting interferons (e.g. albuferon or locteron); antibodies, such as monoclonal antibodies (e.g. XTL-6865, Tarvacin) or polyclonal antibodies (e.g. civacir); immunomodulators, such as immunostimulants (e.g. SCV-07); inhibitors of caspases (e.g. IDN-6556); cyclophilin inhibitors (e.g. Debio-025, SCy-635, NIM-811); alpha-glucosidase I inhibitors (e.g. celgosivir); antisense compounds (e.g. AVI-4065); RNA synthesis inhibitors (e.g. Suvus, Nitazoxanide); and nucleoside analogues (e.g. Ribavarin).

In a further embodiment, combinations of the present invention include a compound of the invention and one or more TLR agonists. Such additional TLR agonists include TLR3 agonists; TLR7 agonists, such as another compound of the invention, ANA-975, SM276001 or *N*-[4-(4-amino-2-ethyl-1*H*-imidazo[4,5-c]quinolin-1-yl)butyl]methanesulfonamide; dual TLR7/TLR8 agonists, such as resiquimod; TLR8 agonists; or TLR9 agonists, such as actilon.

In a further embodiment, combinations of the present invention include a compound of the invention and one or more additional agents useful in the treatment of HIV. Such combinations are useful in the treatment of HCV-HIV co-infection. Agents useful in the treatment of HIV include protease inhibitors; inhibitors of reverse transcriptase, such as NNRTls and NRTls; entry inhibitors such as CCR5 antagonists, agents which inhibit the interaction of gp120 with CD4, and inhibitors of gp41; integrase inhibitors; prenylation inhibitors; RNaseH inhibitors; and maturation inhibitors.

Examples of protease inhibitors include amprenavir; CGP-73547; CGP-61755; mozenavir; nelfinavir; ritonavir; saquinavir; lopinavir; TMC-126; atazanavir, palinavir; GS-3333; KN I-413; KNI-272; LG-71350; CGP-61755; PD 173606; PD 177298; PD 178390; PD 178392; U-140690; ABT-378; DMP-450; AG-1776; MK-944; VX-478; indinavir; tipranavir; TMC-114; DPC-681; DPC-684; fosamprenavir calcium; benzenesulfonamide derivatives disclosed in WO 03/053435; R-944; Ro-03-34649; VX-385; GS-224338; OPT-TL3; PL-100; PPL-100; SM-309515; AG-148; DG-35-VIII; DMP-850; GW-5950X; KNI-1039; L-756423; LB-71262; LP-130; RS-344; SE-063; UIC-94-003; Vb-19038; A-77003; BMS-182193; BMS-186318; SM-309515; JE-2147; and GS-9005

Examples of NNRTls include: efavirenz; HBY-097; nevirapine; TMC-120 (dapivirine); TMC-125, etravirine; delavirdine; DPC-083; DPC-961; capravirine; rilpivirine; 5-{[3,5-Diethyl-1-(2-hydroxyethyl)-1H-pyrazol-4-yl]oxy}isophthalonitrile, or a pharmaceutically acceptable salt or solvate thereof; GW-678248; GW-695634; MIV-150; calanolide; and tricyclic pyrimidinone derivatives as disclosed in WO 03/062238.

Examples of CCR5 antagonists include: TAK-779; SC-351125; ancriviroc; vicriviroc; maraviroc; PRO-140; aplaviroc; AMD-887; CMPD-167; methyl 1-endo-{8-[(3S)-3-(acetylamino)-3-(3-fluorophenyl)propyl]-8-azabicyclo[3.2.1]oct-3-yl}-2-methyl-4,5,6,7-tetrahydro-1H-imidazo[4,5-c]pyridine-5-carboxylate, or a pharmaceutically acceptable salt or solvate thereof; methyl 3-endo-{8-[(3S)-3-(acetamido)-3-(3-fluorophenyl)propyl]-8-azabicyclo[3.2.1]oct-3-yl}-2-methyl-4,5,6,7-tetrahydro-3H-imidazo[4,5-c]pyridine-5-carboxylate, or a pharmaceutically acceptable salt or solvate thereof; ethyl 1-endo-{8-[(3S)-3-(acetylamino)-3-(3-fluorophenyl)propyl]-8-azabicyclo[3.2.1]oct-3-yl}-2-methyl-4,5,6,7-tetrahydro-1H-imidazo[4,5-c]pyridine-5-carboxylate, or pharmaceutically acceptable salt or solvate thereof; and N-{(1S)-3-[3-endo-(5-Isobutyryl-2-methyl-4,5,6,7-tetrahydro-1H-imidazo[4,5-c]pyridin-1-yl)-8-azabicyclo[3.2.1]oct-8-yl]-1-(3-fluorophenyl)propyl}acetamide), or a pharmaceutically acceptable salt or solvate thereof.

Examples of entry and fusion inhibitors include: BMS-806; BMS-488043; 5-{(1S)-2-[(2R)-4-Benzoyl-2-methyl-piperazin-1-yl]-1-methyl-2-oxo-ethoxy}-4-methoxy-pyridine-2-carboxylic acid methylamide; and 4-{(1 S)-2-[(2R)-4-Benzoyl-2-methyl-piperazin-1-yl]-1-methyl-2-oxo-ethoxy}-3-methoxy-N-methyl-benzamide; enfuvirtide (T-20); sifuvirtide SP-01A; T1249; PRO 542; AMD-3100; soluble CD4; compounds disclosed in JP 2003171381; and compounds disclosed in JP 2003119137.

Examples of inhibitors of HIV integrase include: L000870810; GW-810781; 1,5-naphthyridine-3-carboxamide derivatives disclosed in WO 03/062204; compounds disclosed in WO 03/047564; compounds disclosed in WO 03/049690; 5-hydroxypyrimidine-4-carboxamide derivatives disclosed in WO 03/035076; MK-0518; (5-(1,1-dioxo-1,2-thiazinan-2-yl)-N-(4-fluorobenzyl)-8-hydroxy-1,6-naphthyridine-7-carboxamide- disclosed in WO 03016315); 3-(4-fluorobenzyl)-7-hydroxy-1-(piperidin-1-ylmethyl)-3,7,8,9-tetrahydro-6H-pyrrolo[2,3-c]-1,7-naphthyridin-6-one, or pharmaceutically acceptable salt or solvate thereof; and GS-9137 (JTK-303).

Examples of prenylation inhibitors include HMG CoA reductase inhibitors, such as statins (e.g. atorvastatin).

Examples of maturation inhibitors include 3-O-(3'3'-dimethylsuccinyl) betulic acid (otherwise known as PA-457) and alphaHGA.

In a further embodiment, combinations of the present invention include a compound of the invention and one or more additional agents selected from: an antifungal, such as an azole (e.g. fluconazole, fosfluconazole or voriconazole) or a candin (e.g. anidulafungin); an antibacterial, such as a macrolide antibacterial (e.g. azithromycin); an anti-cancer agent, such as an interferon (e.g. interferon alpha), daunorubicin, doxorubicin, or paclitaxel; and an agent to treat cytomegalovirus (CMV) retinitis, such as cidofovir, fomivirsen, foscarnet, ganciclovir or valcyte.

In a further embodiment, combinations of the present invention include a compound of the invention and one or more additional therapeutic agents that enhance the body's immune system. Agents that enhance the body's immune system include low dose cyclophosphamide; thymostimulin; vitamins and nutritional supplement, such as antioxidants (e.g. vitamins A, C, E; beta-carotene; zinc; selenium; glutathione; coenzyme Q-10; and echinacea); and vaccines, such as the immunostimulating complex (ISCOM), which comprises a vaccine formulation that combines a multimeric 5 presentation of antigen and an adjuvant.

Further combinations for use according to the invention include combination of a compound of the invention with a CCR1 antagonist, such as BX-471; a beta adrenoceptor agonist, such as salmeterol; a corticosteroid agonist, such as fluticasone propionate; a LTD4 antagonist, such as montelukast; a muscarinic antagonist, such as tiotropium bromide; a PDE4 inhibitor, such as cilomilast or roflumilast; a COX-2 inhibitor, such as celecoxib, valdecoxib or rofecoxib; an alpha-2-delta ligand, such as gabapentin or pregabalin; a TNF receptor modulator, such as a TNF-alpha inhibitor (e.g. adalimumab); or an immunosuppressant, such as cyclosporin or a macrolide such as tacrolimus.

There is also included within the scope the present invention combinations of a compound of the invention together with one or more additional therapeutic agents which slow down the rate of metabolism of the compound of the invention, thereby leading to increased exposure in patients. Increasing the exposure in such a manner is known as boosting. This has the benefit of increasing the efficacy of the compound of the invention or reducing the dose required to achieve the same efficacy as an unboosted dose. The metabolism of the compounds of the invention includes oxidative processes carried out by P450 (CYP450) enzymes, particularly CYP 3A4 and conjugation by UDP glucuronosyl transferase and sulphating enzymes. Thus, among the agents that may be used to increase the exposure of a patient to a compound of the present invention are those that can act as inhibitors of at least one isoform of the cytochrome P450 (CYP450) enzymes. The isoforms of CYP450 that may be beneficially inhibited include, but are not limited to, CYP1A2, CYP2D6, CYP2C9, CYP2C19 and CYP3A4. Suitable agents that may be used to inhibit CYP 3A4 include ritonavir, saquinavir, ketoconazole, N-(3,4-difluorobenzyl)-N-methyl-2-{[(4-methoxypyridin-3-yl)amino]sulfonyl}benzamide and N-(1-(2-(5-(4-fluorobenzyl)-3-(pyridin-4-yl)-1H-pyrazol-1-yl)acetyl)piperidin-4-yl)methanesulfonamide.

In the above-described combinations, the compound of the invention may be administered simultaneously, sequentially or separately in combination with other therapeutic agent or agents.

It is within the scope of the invention that two or more pharmaceutical compositions, at least one of which contains a compound of the invention, may conveniently be combined in the form of a kit suitable for coadministration of the compositions. Thus the kit of the invention comprises two or more separate pharmaceutical compositions, at least one of which contains a compound of the invention, and means for separately retaining said compositions, such as a container, divided bottle, or divided foil packet. An example of such a kit is the familiar blister pack used for the packaging of tablets, capsules and the like. The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

In another aspect the invention provides a pharmaceutical product (such as in the form of a kit) comprising a compound of the invention together with one or more additional therapeutically active agents as a combined preparation for simultaneous, separate or sequential use in the treatment of a disorder for which a TLR7 agonist is indicated.

It is to be appreciated that all references herein to treatment include curative, palliative and prophylactic treatment.

The invention is illustrated by the following non-limiting examples in which the following abbreviations and definitions are used:
- Arbocel®: Filtration agent, from J. Rettenmaier & Sohne, Germany
- APCI⁺: Atmospheric Pressure Chemical lonisation (positive scan)
- br: Broad
- Chiralpak AD-H: A chromatography column packed with a chiral stationary phase, consisting of amylose tris (3,5-dimethylphenylcarbamate) coated on 5 uM silica gel, from Chrial Technologies Europe
- δ: Chemical shift
- d: Doublet
- DCM: dichloromethane
- dd: Doublet of doublets
- DMSO: Dimethylsulfoxide
- EtOAc: Ethylacetate
- ES+: Electrospray ionisation positive scan
- ¹H NMR: Proton Nuclear Magnetic Resonance Spectroscopy
- HPLC ,: High performance liquid chromatography
- IPA: isopropylalcohol ,
- LC-MS: Liquid Chromatography - Mass Spectrometry
- LRMS: Low Resolution Mass Spectroscopy
- m: Multiplet
- m/z: Mass spectrum peak
- q: Quartet
- s: Singlet
- t: Triplet
- TBME: Tertiary-butyl methyl ether
- THF: Tetrahydrofuran

### Example 1

### 4-Amino-1-(6-methyl-pyridin-3-ylmethyl)-6-(tetrahydro-pyran-4-ylmethoxy)-1,3-dihydro-imidazo[4,5-c]pyridin-2-one

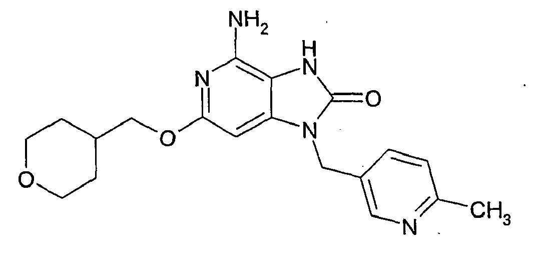

[2,3-Diamino-6-(tetrahydro-pyran-4-ylmethoxy)-pyridin-4-yl]-(6-methyl-pyridin-3-ylmethyl)-carbamic acid ethyl ester (30.5 g) was dissolved in acetic acid (300 ml) and heated at 40 °C for 3 hours. The resultant mixture was then cooled to room temperature and the solvent removed by evaporation under reduced pressure, azeotroping with methanol, to leave a red oil. Diethyl ether (150 ml) was added to provide a suspension that was sonicated and stirred vigorously for 1 hour. The suspension was filtered to give an orange solid. The solid was taken up in methanol (70 ml) and diethyl ether (50 ml) was added. A pale pink precipitate was filtered off and dried in a dessicator. This was then dissolved in approximately 1 L of hot IPA and then allowed to cool to room temperature. The mixture was then cooled to -20 °C for 16 hours. A pale pink solid was filtered off and washed with diethyl ether (200 ml) to provide the title compound (13.2 g) as a pale pink powder.
¹H NMR (DMSO D₆, 400MHz) δ 10.05 (s, 1H), 8.41 (d, 1H), 7.55 (dd, 1H), 7.19 (d, 1H), 5.90 (s, 1H), 5.58 (br, s, 2H), 4.82 (br, s, 2H), 3.90 (q, 2H), 3.85-3.80 (m, 2H), 3.30-3.20 (m, 2H) 2.40 (s, 3H), 1.90-1.80 (m, 1H), 1.65-1.55 (m, 2H), 1.25-1.15 (m, 2H). LC-MS (ES+) 1.32 min, m/z 370 [MH]+

### Example 2

### 4-Amino-1-(6-methyl-pyridin-3-ylmethyl)-6-(tetrahydro-furan-3-R/S-ylmethoxy)-1,3-dihydro-imidazo[4,5-c]pyridin-2-one

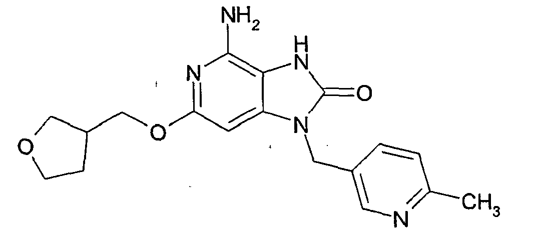

[2,3-Diamino-6-(tetrahydro-furan-3-R/S-ylmethoxy)-pyridin-4-yl]-(6-methyl-pyridin-3-ylmethyl)-carbamic acid ethyl ester (164 mg) was dissolved in acetic acid (5 ml) and heated to 80°C for 2 hours. The resultant mixture was cooled to room temperature and the acetic acid removed in vacuo to leave a brown solid. This solid was taken up into diethyl ether (15 ml) and a brown precipitate formed which was filtered and washed with 3 further portions of diethyl ether (5 ml each). The precipitate was dried in a vacuum oven to give a brown solid. This material was dissolved in methanol, loaded onto silica gel and purified by automated column chromatography, gradient elution (100% DCM to 2% methanol/DCM) providing the title compound (52 mg) as an off white solid.
¹H NMR (CD₃OD 400MHz) δ 8.40 (s, 1H), 7.65 (d, 1H), 7.25 (d, 1H), 5.90 (s, 1H), 5.0 (s, 2H), 4.15-4.05 (m, 1H), 4.05-3.95 (m, 1H), 3.90-3.80 (m, 2H), 3.75-3.70 (m, 1H), 3.65-3.60 (m, 1H) 2.70-2.60 (m, 1H), 2.50 (s, 3H), 2.10-2.00 (m, 1H), 1.75-1.65 (m, 1H). LRMS (ES+) m/z 356 [MH]+

### Example 3

### 4-Amino-1-(6-methyl-pyridin-3-ylmethyl)-6-(tetrahydro-furan-3-S*-ylmethoxy)-1,3-dihydro-imidazo[4,5-c]pyridin-2-one ("S*" indicates absolute, but undefined, stereochemistry)

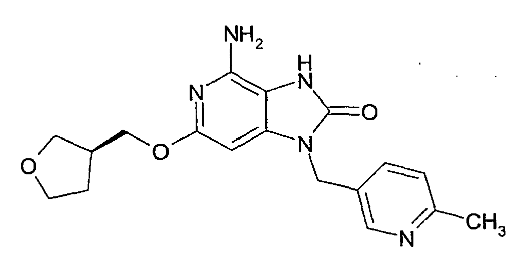

4-Amino-1-(6-methyl-pyridin-3-ylmethyl)-6-(tetrahydro-furan-3-R/S-ylmethoxy)-1,3-dihydro-imidazo[4,5-c]pyridin-2-one (30 mg) was taken up in methanol (5 ml) and the solution passed through a preperative scale chiral HPLC using a solid phase of Chiralpak AD-H, eluting with a 1:1 mixture of methanol:ethanol at a flow rate of 18 ml/min. Product was collected at 7.60 minutes, providing the title compound (10 mg) as a white solid, with >99.5% ee.
¹H NMR (CD₃OD 400MHz) δ 8.40 (s, 1H), 7.65 (d, 1H), 7.25 (d, 1H), 5.90 (s, 1H), 5.0 (s, 2H), 4.15-4.05 (m, 1H), 4.05-3.95 (m, 1H), 3.90-3.80 (m, 2H), 3.75-3.70 (m, 1H), 3.65-3.60 (m, 1H) 2.70-2.60 (m, 1H), 2.50 (s, 3H), 2.10-2.00 (m, 1H), 1.75-1.65 (m, 1H). LRMS (ES+) m/z 356 [MH]+

### Example 4

### 4-Amino-1-(6-methyl-pyridin-3-ylmethyl)-6-(tetrahydro-furan-3-R*-ylmethoxy)-1,3-dihydro-imidazo[4,5-c]pyridin-2-one ("R*" indicates absolute, but undefined, steroechemistry)

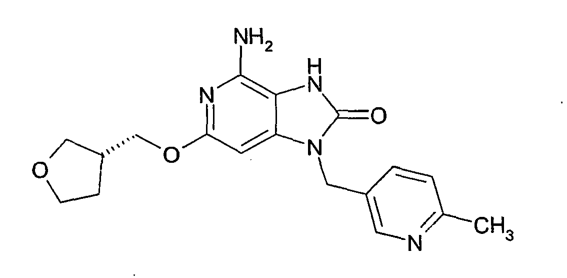

4-Amino-1-(6-methy)-pyridin-3-ylmethyl)-6-(tetrahydro-furan-3-R/S-ylmethoxy)-1,3-dihydro-imidazo[4,5-c]pyridin-2-one (30 mg) was taken up in methanol (5 ml) and the solution passed through a preperative scale chiral HPLC using a solid phase of Chiralpak AD-H, eluting with a 1:1 mixture of methanol:ethanol at a flow rate of 18 ml/min. Product was collected at 8.34 minutes, providing the title compound (11 mg) as a white solid, with 95% ee.
¹H NMR (CD₃OD, 400MHz) δ 8.40 (s, 1H), 7.65 (d, 1H), 7.25 (d, 1H), 5.90 (s, 1H), 5.0 (s, 2H), 4.15-4.05 (m, 1H), 4.05-3.95 (m, 1H), 3.90-3.80 (m, 2H), 3.75-3.70 (m, 1H), 3.65-3.60 (m, 1H) 2.70-2.60 (m, 1H), 2.50 (s, 3H), 2.10-2.00 (m, 1H), 1.75-1.65 (m, 1H). LRMS (ES+) m/z 356 [MH]+

### Example 5

### 4-Amino-1-(6-methyl-pyridin-3-ylmethyl)-6-(tetrahydro-furan-2-R/S-ylmethoxy)-1,3-dihydro-imidazo[4,5-c]pyridin-2-one

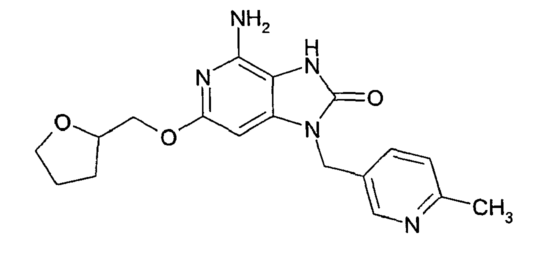

[2,3-Diamino-6-(tetrahydro-furan-2-R/S-ylmethoxy)-pyridin-4-yl]-(6-methyl-pyridin-3-ylmethyl)-carbamic acid ethyl ester (171 mg) was dissolved in acetic acid (5 ml) and heated to 80 °C for 2 hours. The resultant mixture was cooled to room temperature and the acetic acid removed by evaporation under reduced pressure to leave a brown solid. The solid was taken up into diethyl ether (15 ml) and triturated. A brown precipitate formed which was filtered and washed with a further 3 portions of diethyl ether (5 ml each). The precipitate was dried in a vacuum oven to provide the title compound (101 mg) as a brown powder.
¹H NMR (CD₃OD, 400MHz) δ 8.40 (s, 1H), 7.70 (d, 1H), 7.30 (d, 1H), 5.90 (s, 1H), 5.0 (s, 2H), 4.20-4.10 (m, 2H), 4.10-4.05 (m, 1H), 3.90-3.85 (m, 1H), 3.80-3.75 (m, 1H), 2.50 (s, 3H), 2.10-1.85 (m, 3H), 1.75-1.65 (m, 1H). LRMS (ES+) m/z 356 [MH]+

### Example 6

### 4-Amino-1-(6-methyl-pyridin-3-ylmethyl)-6-(tetrahydro-furan-2-R-ylmethoxy)-1,3-dihydro-imidazo[4,5-c]pyridin-2-one

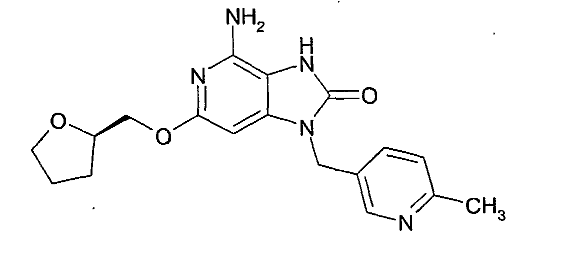

The title compound was prepared using the same method of Example 5, but using enantiopure [2,3-diamino-6-(tetrahydro-furan-2-R-ylmethoxy)-pyridin-4-yl]-(6-methyl-pyridin-3-ylmethyl)-carbamic acid ethyl ester in place of the carbamic acid ethyl ester recited therein.
¹H NMR (CD₃OD, 400MHz) δ 8.40 (s, 1H), 7.70 (d, 1H), 7.30 (d, 1H), 5.90 (s, 1H), 5.0 (s, 2H), 4.20-4.10 (m, 2H), 4.10-4.05 (m, 1H), 3.90-3.85 (m, 1H), 3.80-3.75 (m, 1H), 2.50 (s, 3H), 2.10-1.85 (m, 3H), 1.75-1.65 (m, 1H). LRMS (ES+) m/z 356 [MH]+

### Example 7

### 4-Amino-1-(6-methyl-pyridin-3-ylmethyl)-6-(tetrahydro-furan-2-S-ylmethoxy)-1,3-dihydro-imidazo[4,5-c]pyridin-2-one

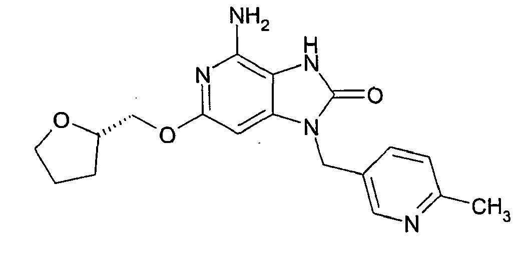

The title compound was prepared using the same method of Example 5, but using enantiopure [2,3-diamino-6-(tetrahydro-furan-2-S-ylmethoxy)-pyridin-4-yl]-(6-methyl-pyridin-3-ylmethyl)-carbamic acid ethyl ester in place of the carbamic acid ethyl ester recited therein.
¹H NMR (CD₃OD 400MHz) δ 8.40 (s, 1H), 7.70 (d, 1H), 7.30 (d, 1H), 5.90 (s, 1H), 5.0 (s, 2H), 4.20-4.10 (m, 2H), 4.10-4.05 (m, 1H), 3.90-3.85 (m, 1H), 3.80-3.75 (m, 1H), 2.50 (s, 3H), 2.10-1.85 (m, 3H), 1.75-1.65 (m, 1H). LRMS (ES+) m/z 356 [MH]+

### Example 8

### 4-Amino-1-(6-methyl-pyridin-3-ylmethyl)-6-tetrahydro-pyran-4-ylmethoxy)-1,3-dihydro-imidazo[4,5-c]pyridin-2-one hydrochloride

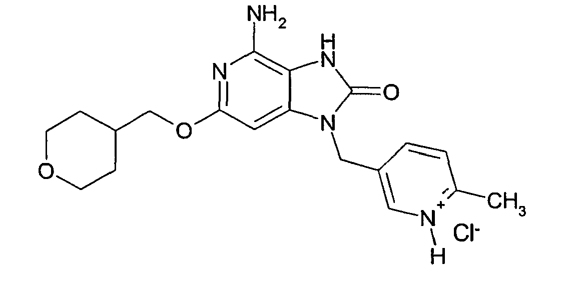

4-Amino-1-(6-methyl-pyridin-3-ylmethyl)-6-(tetrahydro-pyran-4-ylmethoxy)-1,3-dihydro-imidazo[4,5-c]pyridin-2-one (50.0 mg) was suspended in acetonitrile (1.0 ml) and a solution of 1 N HCI (0.2 ml) added. A clear solution was obtained that was stirred for 15 minutes at room temperature, at which time a white solid had precipitated from the mixture. The mixture was stirred for a further 30 minutes and then the solid filtered off. The solid was washed with acetonitrile (0.5ml), dried for a short time on the filter pad, and then dried in a vacuum oven at 50°C. This provided the title compound (20 mg) as a white solid.
¹H NMR (DMSO D₆, 400MHz) δ 8.8 (s, 1H), 8.41 (dd, 1H), 7.85 (d, 1H), 6.60 (s, 1H), 5.30 (s, 2H), 4.15 (d, 2H), 3.95 (m, 2H), 3.45 (m, 2H), 2.79 (s, 3H), 2.15 (m, 1H), 1.75 (m, 2H), 1.45 (m, 2H). LC-MS (ES+) 1.33 min, m/z 370 [MH]+

### Example 9

### 4-Amino-1-(6-methyl-pyridin-3-ylmethyl)-6-(tetrahydro-pyran-4-ylmethoxy)-1,3-dihydro-imidazo[4,5-c]pyridin-2-one (Polymorph Form B)

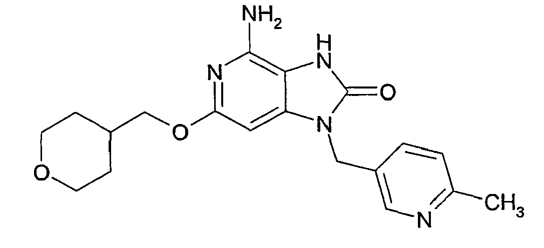

The ethanolic solution of [2,3-diamino-6-(tetrahydropyran-4-ylmethoxy)pyridin-4-yl]-(6-methylpyridin-3-ylmethyl)carbamic acid ethyl ester (323g in 1600mL) from Preparation 22 was charged to the reaction vessel and stirred under nitrogen. Acetic acid (44.5ml) was charged to the vessel and the contents heated at reflux until complete reaction was noted (approximately 2 hours). The reaction mixture was slowly cooled to 5°C and stirred for a further 2hours. The resulting slurry was filtered and washed with ethanol (2x 323mL) to give the crude product as a pink solid. The isolated pink solid was dried under vacuum at 50°C for 15 hours to give 257g, 88%).

The crude material was recharged to the reaction vessel and ethanol (1286ml) charged. The resulting slurry was heated at reflux for 2 hours, cooled to 5°C over 1 hour and stirred at this temperature for a further 2 hours. The pink slurry was filtered and washed with ethanol (2x 128mL) to give a pink solid, which was further dried under vacuum at 50°C for 12 hours to give the title compound (256g, 99%).
¹H NMR (CD3OD D₄, 400MHz) δ 8.40 (s, 1H), 7.65 (dd, 1H), 7.25 (d, 1H), 5.90 (s, 1H), 4.95 (s, 2H), 3.90 (m, 4H), 3.40 (m, 2H) 2.50 (s, 3H), 2.05-1.90 (m, 1H), 1.70 (m, 2H), 1.38 (m, 2H).

PXRD analysis, as described hereinafter, showed the title compound to be a single polymorph, designated Form B.

### Example 10.

### 4-Amino-1-(6-methyl-pvridin-3-ylmethyl)-6-(tetrahydro-pyran-4-ylmethoxy)-1,3-dihydro-imidazo[4,5-c]pyridin-2-one (Polymorph Form A)

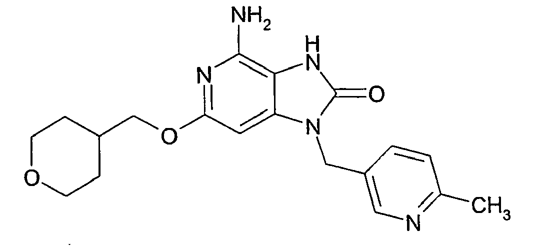

A solution of [2,3-diamino-6-(tetrahydropyran-4-ylmethoxy)pyridin-4-yl]-(6-methylpyridin-3-ylmethyl)carbamic acid ethyl ester (405mg) in ethanol (4.5mL) and acetic acid (0.5mL) was heated at 80°C until complete reaction is noted (approximately 2 hours). The reaction mixture was concentrated under reduced pressure, azeotroping with methanol. The resultant brown solid was dissolved in methanol (approx. 10mLs) and refrigerated overnight. The resulting slurry was filtered, washed with methanol and dried under vacuum at 40°C for 2 hours to give 257g (88%) of the title compound as an off-white solid.
¹H NMR (DMSO D₆, 400MHz) δ 10.05 (s, 1H), 8.41 (d, 1H), 7.55 (dd, 1H), 7.19 (d, 1H), 5.90 (s, 1H), 5.58 (br, s, 2H), 4.82 (br, s, 2H), 3.90 (q, 2H), 3.85-3.80 (m, 2H), 3.30-3.20 (m, 2H) 2.40 (s, 3H), 1.90-1.80 (m, 1H), 1.65-1.55 (m, 2H), 1.25-1.15 (m, 2H). LC-MS (ES+) 1.32 min, m/z 370 [MH]+

PXRD analysis, as described hereinafter, showed the title compound to be a single polymorph, designated Form A.

### Preparation 1

### 2,6-Dichloro-4-(N-nitro)amino-pyridine

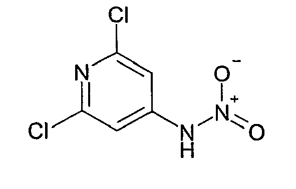

2,6-Dichloro-4-amino pyridine (43.8 g) was taken up in sulfuric acid (620 ml) at 0 °C under a nitrogen atmosphere and nitric acid (12 ml) added drop-wise over 1 hour at such a rate that the temperature did not go above 0 °C. Once all the nitric acid had been added, the red/orange solution was stirred at 0 °C for 1 hour then poured carefully onto crushed ice (2.4 L) with stirring. The precipitate was collected by filtration then re-suspended in water (1 L) and filtered once more. The solid was left to dry in a desiccator over P₂O₅ overnight, yielding the title compound (52.83 g) as an off-white solid.
¹H NMR (CDCl₃, 400MHz) δ 10.4 (s, 1H), 7.40 (s, 2H). LC-MS (AP+) 2.56 min, m/z 209 [MH]+

### Preparation 2

### 2,6-Dichloro-4-amino-5-nitro-pyridine

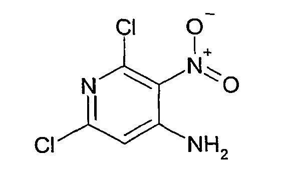

Sulfuric acid (550 mL) was heated to 50 °C then the heat bath removed. 2,6-Dichloro-4-(N-nitro)aminopyridine (52.83g) was added portion-wise over 45 minutes to the warmed sulfuric acid at such a rate that the temperature of the reaction mixture remained between 46 and 48 °C. On complete addition the reaction was warmed to 50 °C once more. After 30 min the resultant mixture was allowed to cool to room temperature then poured slowly onto crushed ice (3 L) with vigorous stirring. The precipitate was collected by filtration then suspended in water (1 L) and re-filtered. The solid was then dissolved in ethyl acetate (400 mL), transferred to a separating funnel and the residual aqueous phase removed before washing the remaining organic phase with water (100 mL), saturated sodium bicarbonate solution (100 mL), and brine (100 mL). The organic phase was then dried (MgSO₄) and the solvent removed under reduced pressure to afford the title compound (34.82 g) as a pale yellow solid.
¹H NMR (CDCI₃, 400MHz) δ 6.70 (s, 1H), 5.70 (s, 2H). LRMS (ES+) m/z 209 [MH]+

### Preparation 3

### (2,6-Dichloro-3-nitro-pyridin-4-yl)-carbamic acid ethyl ester

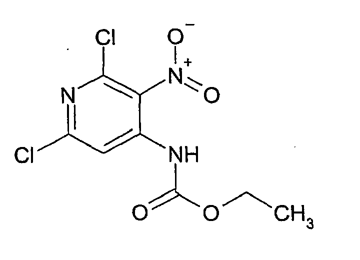

A solution of ethyl chloroformate (17.4 ml) in anhydrous 2-methyl THF (50 ml) was added drop-wise to a cooled (0 °C) solution of 2,6-dichloro-4-amino-5-nitro-pyridine (34.5 g) and triethylamine (46 ml) in anhydrous 2-methyl THF (450 ml) at 0 °C over 1 hour, keeping the addition rate such that the reaction temperature did not rise above 5 °C. The resulting turbid, bright yellow, solution was stirred at 0 °C for 45 minutes and then allowed to warm to room temperature. After a further 2 hours stirring at room temperature, water (200 ml) was added to quench the reaction and the mixture transferred to a separating funnel. The layers were separated and the aqueous extracted with ethyl acetate (3 x 100 mL), and the combined extracts washed with brine (100 ml), and dried (MgSO₄) Removal of the solvent then afforded a viscous orange oil from which a solid compound crystallised out over 2 days. The crystals were filtered from the mixture, washed with cold methanol (3 x 25 mL) and dried by evaporation under reduced pressure to afford the title compound as clear pale yellow crystals (12.43 g). The filtrates were combined and concentrated to afford a dark orange oil. The oil was purified by automated column chromatography (SiO₂; gradient elution 10 to 30% ethyl acetate in pentane) to afford a second batch of title compound as a yellow solid (4.07 g), along with a third batch (21.94. g). All batches were combined to afford the title compound (36.51 g) as a yellow crystalline solid.
¹H NMR (CDCI₃, 400MHz) δ 8.40 (s, 1H), 8.10 (br, s, 1H), 4.30 (q, 2H), 1.35 (t, 3H).
LC-MS (ES+) 2.98 min, m/z 280 [MH]+

### Preparation 4

### (2,6-Dichloro-3-nitro-pyridin-4-yl)-(6-methyl-pyridin-3-ylmethyl)-carbamic acid ethyl ester

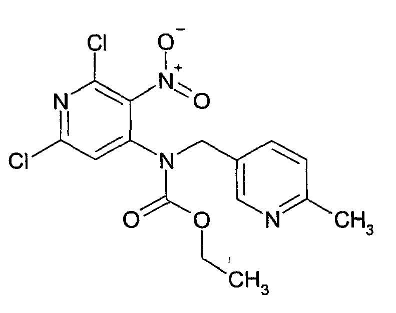

Potassium carbonate (25.1 g) was added to a solution of (2,6-dichloro-3-nitro-pyridin-4-yl)-carbamic acid ethyl ester (25.5 g) and 5-(chloromethyl)-2-methylpyridine (12.9 g) in acetone (365 ml) at room temperature under nitrogen. The solution turned dark brown and was stirred for 5 minutes. Sodium iodide (16.4 g) was added in one portion and the reaction mixture stirred for three days at room temperature. The solvent was removed by evaporation under reduced pressure to leave a brown solid. The crude material was taken up into ethyl acetate (500 ml) and water (500 ml) added. The phases were separated and the aqueous phase was further extracted with ethyl acetate (2 x 250 ml). The combined organic fractions were washed with water (2 x 250 ml), dried (MgSO₄), filtered and the solvent removed by evaporation under reduced pressure to leave a dark brown gum. This was purified by column chromatography (SiO₂, eluting with 20% ethyl acetate in heptane and increasing to 40 % ethyl acetate in heptane) to obtain the title compound (22.8 g) as a green oil.
¹H NMR (CDCl₃, 400MHz) δ 8.40 (s, 1H), 7.60 (d, 1H), 7.20 (d, 1H), 7.00 (s, 1H), 4.80 (s, 2H), 4.25 (q, 2H), 2.58 (s, 3H), 1.25 (t, 3H). LC-MS (ES+) 2.12 min, m/z 385 [MH]+

### Preparation 5

### Benzyl-(2,6-dichloro-3-nitro-pyridin-4-yl)-carbamic acid ethyl ester

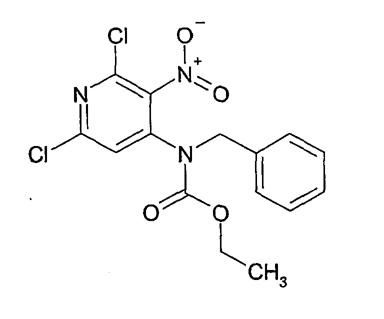

Benzyl bromide (2.33 ml) was added drop-wise to a stirred suspension of (2,6-dichloro-3-nitro-pyridin-4-yl)-carbamic acid ethyl ester (4.57 g) and potassium carbonate (4.51 g) in acetonitrile (40 ml) and the reaction mixture left to stir at room temperature under nitrogen for 16 hours. The mixture was concentrated *in vacuo* then partitioned between ethyl acetate (50 ml) and water (50 ml). The layers were separated and the organics were washed with saturated NH₄Cl (50 ml), water (50 ml) and brine (50 ml). Combined organics were dried (MgSO₄) and evaporated under reduced pressure to give a yellow oil. This was adsorbed onto silica and purified by automated column chromatography on a silica column (330 g, Redisep), eluting with ethyl acetate:heptane, isocratic at 10:90 for 1 column volume then increasing the gradient from 10:90 to 30:70 over 6 column volumes. The desired fractions were combined and evaporated to provide the title compound (5.96 g) as a yellow oil.
¹H NMR (CDCl₃, 400MHz) δ 7.40-7.30 (m, 3H), 7.25-7.20 (m, 2H), 6.88 (s, 1H), 4.80 (s, 2H), 4.20 (q, 2H), 1.25 (t, 3H). LC-MS (ES+) 3.62 min, m/z 370 [MH]+

### Preparation 6

### (2-Amino-6-chloro-3-nitro-pyridin-4-yl)-(6-methyl-pyridin-3-ylmethyl)-carbamic acid ethyl ester

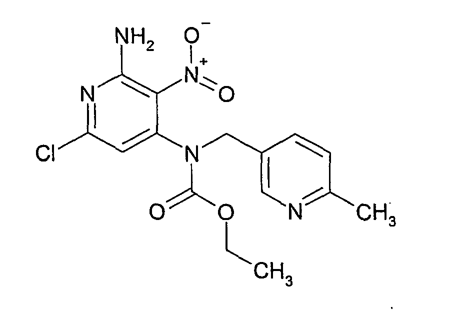

Ammonia (325 ml) was added to a solution of (2,6-dichloro-3-nitro-pyridin-4-yl)-(6-methyl-pyridin-3-ylmethyl)-carbamic acid ethyl ester (50.1 g) in 2-methyl-THF (325 ml). The reaction vessel was sealed and the solution stirred at room temperature for 20 hours. Ethyl acetate (500 ml) and water (500 ml) were added and the phases separated. The aqueous phase was further extracted with ethyl acetate (2 x 250 ml) and the combined extracts were dried (MgSO₄), filtered and the solvent removed by evaporation under reduced pressure to leave an orange foam. TBME (50 ml) was added and the solvent removed to leave a dark yellow solid. The solid was slurried in hot TBME (80 ml) and stirred under reflux for 30 minutes. The mixture was cooled to room temperature and the solid filtered to leave a yellow powder. This process was repeated 3 times to provide a yellow powder (37 g) and combined filtrates which were evaporated under reduced pressure to leave a brown oil (11 g). The solid was then split into 3 batches (1 x 10 g, 1 x 12 g, 1 x 15 g) and for each batch the following, procedure was carried out: The material was dissolved in a mixture of refluxing 80% DCM 20% acetone (approx 100 ml). The solution was passed through a plug of silica in a sinter eluting with 80 % DCM 10% acetone 10% heptane (10 1) until all of the visible yellow band was collected. The solvent was removed by evaporation under reduced pressure to provide 3 batches of a yellow foam. The combined filtrates (11 g) were purified by automated column chromatography eluting with 80 % DCM 10% acetone 10% heptane to give a further batch of product as a yellow solid. All batches were combined to provide the title compound (40 g) as a yellow solid.
¹H NMR (Acetone D₆, 400MHz) δ 8.45 (s, 1H), 7.65 (d, 1H), 7.21 (d, 1H), 7.18 (br, s, 2H), 6.70 (s, 1H), 5.00 (br, s, 2H), 4.15 (br, q, 2H), 2.42 (s, 3H), 1.18 (br, t, 3H). LC-MS (ES+) 0.98 min, m/z 366 [MH]+

### Preparation 7

### (2-Amino-6-chloro-3-nitro-pyridin-4-yl)-benzvl-carbamic acid ethyl ester

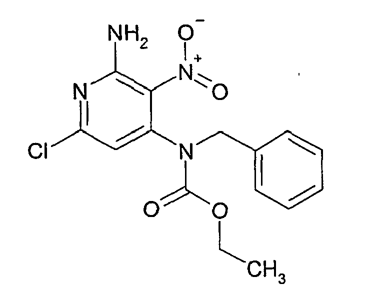

Ammonia (7M in methanol, 1 ml) was added to a solution of benzyl-(2,6-dichloro-3-nitro-pyridin-4-yl)-carbamic acid ethyl ester (500 mg) in 2-methyl-THF (3 ml). The reaction vessel was sealed and the solution stirred at room temperature for 48 hours. The reaction mixture was adsorbed onto silica and purified by automated column chromatograhpy on a silica column (40 g, Redisep), eluting with ethyl acetate:heptane, increasing the gradient linearly from 10:90 to 40:60 over 10 column volumes. The desired fractions were combined and evaporated to a yellow gum which solidified on scratching, thus providing the title compound (304 mg) as a yellow solid.
¹H NMR (DMSO D₆, 400MHz) δ 7.60 (br, s, 2H), 7.35-7.20 (m, 5H), 6.59 (s, 1H), 4.85 (br, s, 2H), 4.00 (q, 2H), 1.05 (t, 3H). LC-MS (ES+) 3.24 min, m/z 351 [MH]+

### Preparation 8

### [2-Amino-3-nitro-6-(tetrahydro-4)-pyran-4-ylmethoxy)-pyridin-4-yl]-(6-methyl-pyridin-3-ylmethyl)-carbamic acid ethyl ester

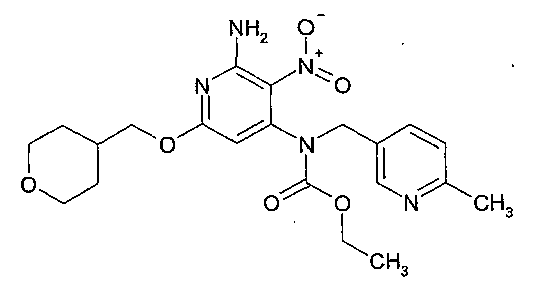

Sodium hydride (60% dispersion in mineral oil, 7.52 g) was added portion-wise to a solution of (tetrahydro-pyran-4-yl)-methanol (21.8 g) in THF (350 ml) at room temperature under nitrogen and the resulting suspension was stirred for 30 minutes. A solution of (2-amino-6-chloro-3-nitro-pyridin-4-yl)-(6-methyl-pyridin-3-ylmethyl)-carbamic acid ethyl ester (34.0 g) in THF (150 ml) was then added and the deep red reaction mixture stirred for 16 hours at room temperature. Solvent was removed by evaporation under reduced pressure and the resulting gum partitioned between ethyl acetate (750 ml) and water (500 ml). Brine (100 ml) was added and the phases separated. The organic phase was collected and the aqueous washed with ethyl acetate (2 x 250 ml). The combined organics were then dried (MgSO₄) and the solvent removed in vacuo to give an orange oil. The oil was taken up into DCM (250 ml) and the solvent removed to leave an orange foam. TBME (250 ml) was added and the solvent removed under reduced pressure to give an orange solid. The solid was slurried in hot TBME (150 ml) and stirred at reflux for 30 minutes. The suspension was cooled to room temperature and filtered to provide the title compound (35.3 g) as an orange powder.
¹H NMR (DMSO D₆, 400MHz) δ 8.30 (s, 1H), 7.75 (br, s, 2H), 7.48 (d, 1H), 7.15 (d, 1H), 5.90 (s, 1H), 4.90-4.60 (br, d, 2H), 4.10 (d, 2H), 4.05 (br, q, 2H), 3.80 (d, 2H), 3.30-3.20 (m, 2H) 2.40 (s, 3H), 1.95-1.85 (m, 1H), 1.60-1.50 (m, 2H), 1.30-1.20 (m, 2H), 1.05 (br, t, 3H). LRMS (ES+) m/z 446 [MH]+

### Preparation 9

### [2,3 Diamino-6-(tetrahydro-pyran-4-ylmethoxy)-pyridin-4-yl)-(6-methyl-pyridin-3-ylmethyl)-carbamic acid ethyl ester

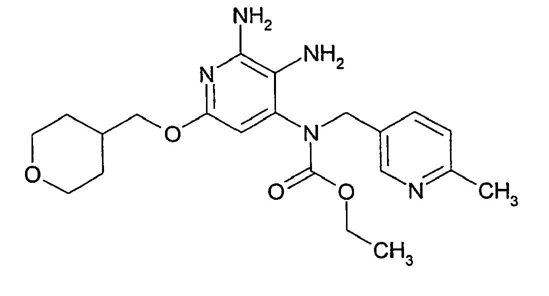

[2-Amino-3-nitro-6-(tetrahydro-pyran-4-ylmethoxy)-pyridin-4-yl]-(6-methyl-pyridin-3-ylmethyl)-carbamic acid ethyl ester (20.0 g) was taken up in ethanol (800 ml) and heated to 40 °C for 30 minutes. The majority of the material dissolved. Pd/C (4.00 g) was added and the mixture was heated to 40 °C under 40 psi of hydrogen for 4 hours. The green solution was filtered through arbocel and the filter cake washed with ethanol (∼1.5 L) until most of the colour was washed through. The solvent was removed by evaporation under reduced pressure to leave a yellow/brown foam. The material was re-dissolved in ethanol (200 ml) and the material was filtered through celite to remove residual Pd catalyst. The filter cake was washed with methanol (200 ml) until the colour was washed through. The solvents were removed by evaporation under reduced pressure to provide the title compound (15.6 g) as a brown oil that was used in the next step without further purification.
LC-MS (ES+) 0.72 min, LRMS m/z 416 [MH]+

### Preparation 10

### [2-Amino-3-nitro-6-(tetrahydro-furan-3-R/S-ylmethoxy)-pyridin-4-yl]-(6-methyl-pyridin-3-ylmethyl)-carbamic acid ethyl ester

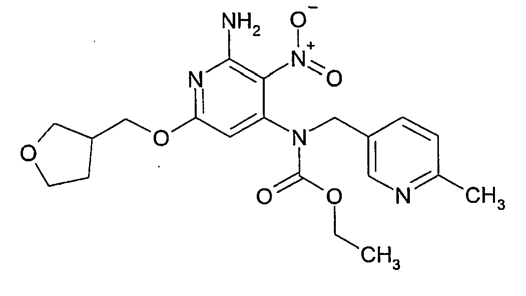

Sodium hydride (55 mg) was added to a solution of (R/S-tetrahydro-furan-3-yl)-methanol (79 ul) in THF (10 ml) and the resulting cloudy mixture was stirred at room temperature for 5 minutes. (2-Amino-6-chloro-3-nitro-pyridin-4-yl)-(6-methyl-pyridin-3-ylmethyl)-carbamic acid ethyl ester (200 mg) was added and the deep red mixture stirred at room temperature for 3 hours. Water (10 ml) and brine (10 ml) were added and the mixture extracted into ethyl acetate (3 x 20 ml). Combined organic fractions were dried (Na₂SO₄), filtered and the solvent removed by evaporation under reduced pressure to leave a brown oil. The crude material was loaded onto silica gel and purified by column chromatography (gradient elution 20% ethyl acetate in pentane to 100% ethyl acetate over 30 minutes) providing the title compound (220 mg) as a yellow foam.
¹H NMR (CDCl₃, 400MHz) δ 8.35 (s, 1H), 7.60 (br, s, 1H), 7.09 (d, 1H), 6.60 (br, s, 2H), 5.78 (s, 1H), 4.95 (br, d, 1H), 4.50 (br, d, 1H), 4.20-4.00 (m, 4H), 3.85-3.75 (m, 2H), 3.74-3.65 (m, 1H), 3.60-3.50 (m, 1H) 2.65-2.55 (m, 1H), 2.45 (s, 3H), 2.05-1.95 (m, 2H), 0.90 (br, t, 3H). LRMS (ES+) m/z 432 [MH]+

### Preparation 11

### [2,3-Diamino-6-(tetrahydro-furan-3-R/S-ylmethoxy)-pyridin-yl]-(6-methyl-pyridin-3-ylmethyl)-carbamic acid ethyl ester

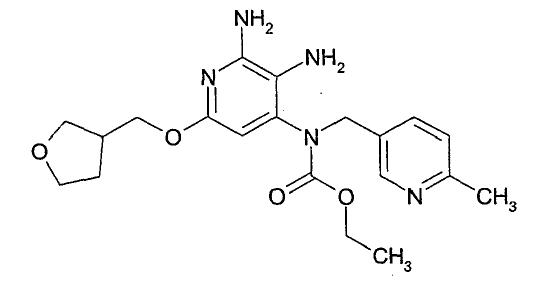

Pd/C (22 mg) was added to a solution of [2-amino-3-nitro-6-(tetrahydro-furan-3-R/S-ylmethoxy)-pyridin-4-yl]-(6-methyl-pyridin-3-ylmethyl)-carbamic acid ethyl ester (220 mg) in ethanol (10 ml). The reaction mixture was heated at 40 °C under 40 psi hydrogen for 2 hours. The mixture was filtered through arbocel and the solvent removed by evaporation under reduced pressure to provide the title compound (202 mg) as a green oil that was used without further purification.
LC-MS (ES+) 0.69 min, LRMS m/z 402 [MH]+

### Preparation 12

### [2-Amino-3-nitro-6-(tetrahydro-furan-2-R/S-ylmethoxy)-pyridin-4-yl]-(6-methyl-pyridin-3-yl-methyl)-carbamic acid ethyl ester

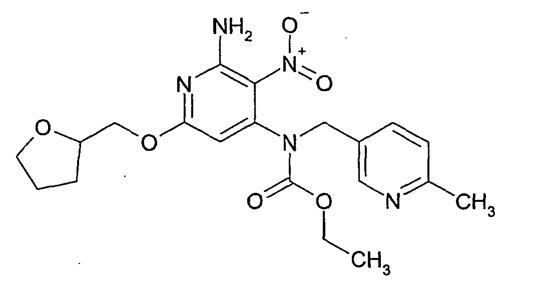

Sodium hydride (87.5 mg) was added to a solution of (R/S-tetrahydro-furan-2-yl)-methanol (159 ul) in THF (10 ml) and the resulting cloudy mixture was stirred at room temperature for 5 minutes. (2-Amino-6-chloro-3-nitro-pyridin-4-yl)-(6-methyl-pyridin-3-ylmethyl)-carbamic acid ethyl ester (200 mg) was added and the mixture turned a deep red with effervescence observed. The resultant mixture was stirred at room temperature for 3 hours. Water (10 ml) and brine (10 ml) were added and the mixture extracted into ethyl acetate (3 x 20 ml). Combined organic fractions were dried (Na₂SO₄), filtered and the solvent removed by evaporation under reduced pressure to leave a brown oil. The crude material was loaded onto silica gel and purified by automated column chromatography (gradient elution 40% pentane/ethyl acetate to 100% ethyl acetate over 30 minutes) providing the title compound (169 mg) as a yellow foam.
¹H NMR (CD₃OD, 400MHz) δ 8.30 (s, 1H), 7.65 (d, 1H), 7.10 (d, 1H), 5.85 (br, s, 1H), 5.00-4.95 (br, d, 1H), 4.65-4.60 (br, d, 1H), 4.30-4.25 (m, 1H), 4.20-4.00 (m, 4H), 3.90-3.70 (m, 2H), 2.45 (s, 3H), 2.05-1.95 (m, 1H), 1.95-1.85 (m, 2H), 1.65-1.60 (m, 1H), 1.10 (br, t, 3H).
LC-MS (ES+) 1.28 min, LRMS (ES+) m/z 432 [MH]+

### Preparation 13

### [2,3-Diamino-6-(tetrahydro-furan-2-R/S-ylmethoxy)-pyridin-4-yl]-(6-methyl-pyridin-3-ylmethyl)-carbamic acid ethyl ester

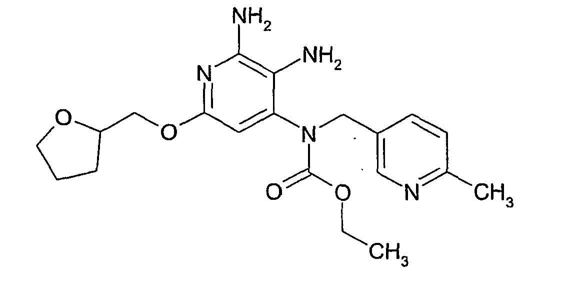

Pd/C (22 mg) was added to a solution of [2-amino-3-nitro-6-(tetrahydro-furan-2-RIS-ylmethoxy)-pyridin-4-yl]-(6-methyl-pyridin-3-ylmethyl)-carbamic acid ethyl ester (220 mg) in ethanol (5 ml). The resultant mixture was heated to 40 °C under 40 psi of hydrogen for 90 minutes. After cooling to room temperature, the mixture was filtered through arbocel and the solvent removed by evaporation under reduced pressure to provide the title compound (173 mg) as a yellow foam which was used in the next step without further purification.
LC-MS (ES+) 1.81 min, LRMS m/z 402 [MH]+

### Preparation 14

### [2-Amino-3-nitro-6-(tetrahydro-furan-2-R-ylmethoxy)-pyridin-4-yl]-(6-methyl-pyridin-3-ylmethyl)-carbamic acid ethyl ester

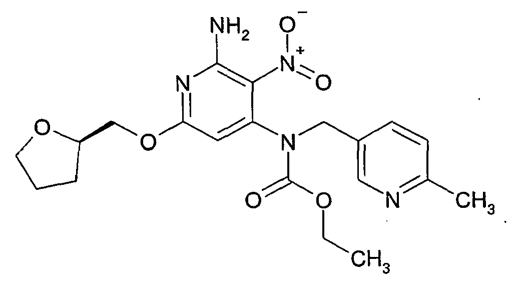

The title compound was prepared in analogous fashion to that illustrated in Preparation 12, using in this case enantiopure (R-tetrahydro-furan-2-yl)-methanol.
LRMS (ES+) m/z 432 [MH]+

### Preparation 15

### [2,3-Diamino-6-(tetrahydro-furan-2-R-ylmethoxy)-pyridin-4-yl]-(6-methyl-pyridin-3-ylmethyl)-carbamic acid ethyl ester

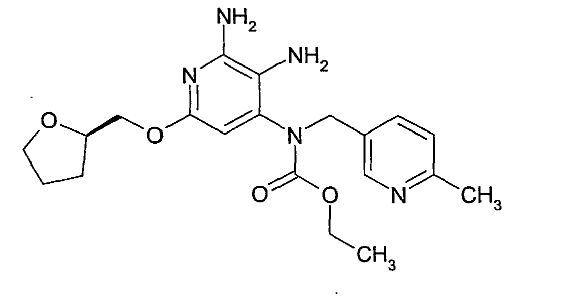

The title compound was prepared in analogous fashion to that illustrated in Preparation 13, using in this case enantiopure [2-amino-3-nitro-6-(tetrahydro-furan-2-R-ylmethoxy)-pyridin-4-yl]-(6-methyl-pyridin-3-ylmethyl)-carbamic acid ethyl ester.
LRMS m/z 402 [MH]+

### Preparation 16

### [2-Amino-3-nitro-6-(tetrahydro-furan-2-S-ylmethoxy)-pyridin-4-yl]-(6-methyl-pyridin-3-ylmethyl)-carbamic acid ethyl ester

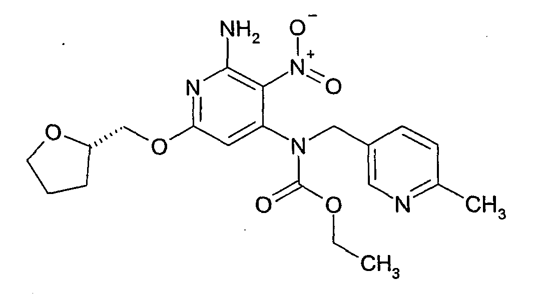

The title compound was prepared in analogous fashion to that illustrated in Preparation 12, using in this case enantiopure (S-tetrahydro-furan-2-yl)-methanol.
LRMS (ES+) m/z 432 [MH]+

### Preparation 17

### [2,3-Diamino-6-(tetrahydro-furan-2-S-ylmethoxy)-pyridin-4-yl]1-(6-methyl-pyridin-3-ylmethyl)-carbamic acid ethyl ester

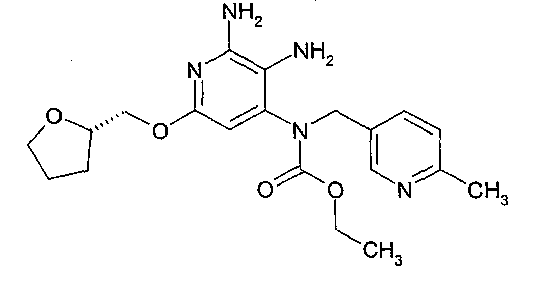

The title compound was prepared in analogous fashion to that illustrated in Preparation 13, using in this case enantiopure [2-amino-3-nitro-6-(tetrahydro-furan-2-S-ylmethoxy)-pyridin-4-yl]-(6-methyl-pyridin-3-ylmethyl)-carbamic acid ethyl ester.
LRMS m/z 402 [MH]+

### Preparation 18

### (2,6-Dichloro-3-nitro-pyridin-4-yl)-carbamic acid ethyl ester

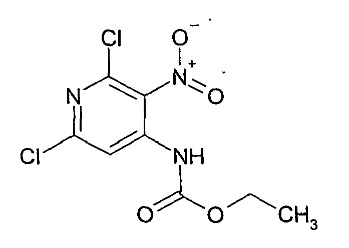

A 1 M solution of potassium t-butoxide (2.64mol, 297g) in THF (2.64L) was added dropwise to a cooled (-20°C) solution of 6-dichloro-4-amino-5-nitro-pyridine (500g) in THF (2.5L), keeping the addition rate such that the internal reaction temperature was maintained between -20°C and -15°C. The resulting red suspension was stirred at -20°C to -15°C for 1 hour. A solution of ethyl chloroformate (313g) in THF (1000ml) was added slowly to the mixture over 1 hour, maintaining the internal temperature between -20°C and -15 °C. The resulting brown suspension was stirred between -20°C and -15 °C for 30 mins. A 1 M solution of potassium t-butoxide (4.57mol, 512g) in THF (4.6L) was prepared and added slowly over 3hours to the reaction mixture, again keeping the internal temperature between -20°C and -15 °C. The resulting dark brown suspension was warmed to 20°C over 1 hour and maintained at this temperature for 2hrs. The reaction mixture was cooled to 5oC and 1 M aqueous citric acid (5L) was added slowly to the reaction mixture, maintaining the internal temperature below 20°C. The resulting biphasic mixture was stirred at 20°C for 1h and washed with EtOAc (2.5L). The phases were separated and the organic phase was washed with saturated aqueous NaHCO₃ solution (5L), followed by washing with saturated aqueous NaCl solution (5L). The organic layer was separated and concentrated to ∼1L (2ml/g) at 40°C under reduced pressure (∼250 mbar) to give the title compound as a solution in EtOAc. Acetone (9.0L, 18ml/g) was added and the mixture used directly in Preparation 19.
¹H NMR (CD₃OD D₄, 400MHz) δ 8.24 (s, 1H), 4.25 (q, 2H), 1.30 (t, 3H).

### Preparation 19

### (2,6-Dichloro-3-nitro-pyridin-4-yl)-(6-methyl-pyridin-3-ylmethyl)-carbamic acid ethyl ester

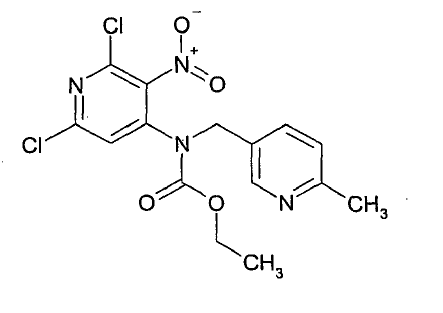

Potassium carbonate (663.4g) was added to the solution from Preparation 19 of (2,6-Dichloro-3-nitropyridin-4-yl)carbamic acid ethyl ester in EtOAc:acetone (∼672g in 10L EtOAc:acetone) at 20°C under nitrogen. Sodium iodide (1080g) was added to the stirred solution at 20°C, followed by 5-(chloromethyl)-2-methylpyridine hydrochloride (427.3g). The resulting orange suspension was heated to 50°C and stirred at this temperature for 4hrs. The reaction mixture was cooled to 5°C, stirred at this temperature for 1 hr and then filtered. The filter cake was washed with acetone (2mUg) and the combined organic extracts evaporated to dryness under reduced pressure at 40°C to give a dark brown gummy solid. Dichloromethane (1613ml), 2.4ml/g) was added to the solid and the resulting slurry stirred for 1 hr to dissolve. This solution was purified by automated column chromatography (Biotage silica cartridge, 150L, 5 Kg of silica, CV=8.6 L) eluting with toluene : EtOAc 2:1. The desired fractions were combined and evaporated to give the title compound as a dark purple oil (592g, 64%).

### Preparation 20

### (2-Amino-6-chloro-3-nitro-pyridin-4-yl)-(6-methyl-pyridin-3-ylmethyl)-carbamic acid ethyl ester

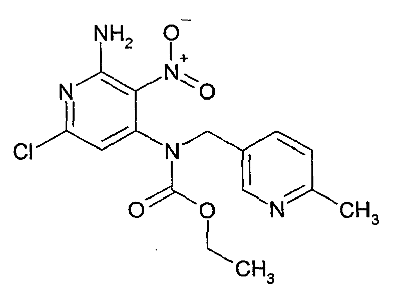

Ammonium hydroxide solution (770ml) was charged to a stirred solution of (2,6-dichloro-3-nitro-pyridin-4-yl)-(6-methylpyridin-3-ylmethyl)carbamic acid ethyl ester (592g) in 2-methyl-THF (2000ml) at ambient temperature. The reaction mixture was stirred at ambient temperature for 6 hours, a further portion of ammonium hydroxide solution (770ml) was charged at ambient temperature and the reaction, mixture stirred for a further 15 hours. A third portion of ammonium hydroxide solution (770mL) was charged to the reaction vessel at ambient temperature and the reaction stirred for a further 4 hours. On reaction completion, the organic layer was separated and washed with 20% aqueous sodium chloride solution (3000mL). The organic layer was separated and concentrated to 1000mL at 35°C under reduced pressure. TBME (7500mL) was charged and the procedure repeated twice more. The resulting thick slurry was cooled to 5°C and stirred at this temp for 60 minutes, filtered and washed with TBME (300mL). The isolated yellow solid was further dried under vacuum at 50°C for 15 hours to give the title compound (423g, 75%)
¹H NMR (CDCl₃, 400MHz) δ 8.40 (s, 1H), 7.60 (br, s, 1H), 7.15 (d, 1H), 6.40 (s, 3H), 5.00-4.70 (br, m, 2H), 4.15 (br, m, 2H), 2.55 (s, 3H), 1.20 (br, m, 3H).

### Preparation 21

### [2-Amino-3-nitro-6-(tetrahydro-pyran-4-ylmethoxy)-pyridin-4-yl]-(6-methyl-pyridin-3-ylmethyl)-carbamic acid ethyl ester

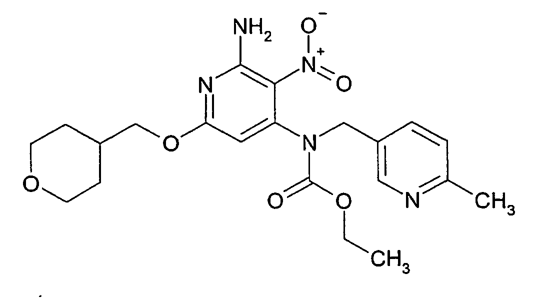

A solution of (2-amino-6-chloro-3-nitropyridin-4-yl)-(6-methylpyridin-3-ylmethyl)-carbamic acid ethyl ester (400g) and (tetrahydropyran-4-yl)methanol (152g) in tetrahydrofuran (2000mL) was charged slowly to a suspension of sodium hydride (96g) in tetrahydrofuran (2000mL) at 0-5°C over 1 hour maintaining the internal temperature at 0-5°C. The resulting red mixture was stirred at 0-5°C for 1 hour and allowed to warm to ambient temperature over 15-30 minutes. The reaction mixture was stirred at ambient temperature until complete. The reaction mixture was quenched by slow addition of water (400mL), sodium chloride solution (20%w/v, 3000mL) was charged and the contents stirred for 15-20 minutes and the layers separated. The aqueous phase was extracted with ethyl acetate (2000mL) and the organic phases combined and concentrated under reduced pressure to 800mL. TBME (5000mL) was charged and the resulting mixture re-concentrated to 800mL under reduced pressure. This process was repeated twice more. The resulting orange slurry was cooled to 0-10°C, stirred for 2 hours, filtered, washed with TBME (200mL). The isolated orange solid was further dried under vacuum at 50°C for 15 hours to give the title compound (421g, 86%).

The isolated orange solid was recrystallised in refluxing 2-propanol (3368mL), cooled to ambient temperature, filtered and dried under vacuum at 50°C for 16 hours to give 365g (87% recovery).
¹H NMR (DMSO D₆, 400MHz) δ 8.30 (s, 1H), 7.80 (br, s, 2H), 7.58 (d, 1H), 7.15 (d, 1H), 5.95 (s, 1H), 4.95-4.60 (br, d, 2H), 4.10 (d, 2H), 4.00 (br, q, 2H), 3.82 (d, 2H), 3.35-3.25 (m, 2H) 2.40 (s, 3H), 1.95 (m, 1H), 1.55 (m, 2H), 1.25 (m, 2H), 1.05 (br, m, 3H).

### Preparation 22

### [2,3-Diamino-6-(tetrahydro-pyran-4-ylmethoxy)-pyridin4-yl]-(6-methyl-pyridin-3-ylmethyl)-carbamic acid ethyl ester

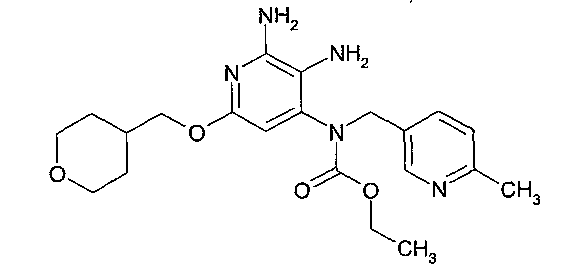

2-Amino-3-nitro-6-(tetrahydropyran-4-ylmethoxy)pyridin-4-yl]-(6-methylpyridin-3-ylmethyl)carbamic acid ethyl ester (365g) was dissolved in methanol (7300ml) and stirred with 10% Pd(OH)₂ on carbon (37g) under an atmosphere of hydrogen (20psi) at 40°C for 3 hours. The reaction mixture was filtered, washed with methanol (2x 1100mL) and the liquors concentrated under reduced pressure at to 40°C to 700mL. Ethanol (3700mL) was charged and the mixture re-concentrated under reduced pressure at to 40°C to 700mL. This procedure was repeated once more and ethanol (900mL) charged to give a final ethanolic solution (1600mL) of the title compound which was used directly in Example 9.

### Polymorphs of 4-Amino-1-(6-methyl-pyridin-3-ylmethyl)-6-(tetrahydro-pyran-4-ylmethoxy)-1,3-dihydro-imidazo[4,5-c]pyridin-2-one

4-Amino-1-(6-methyl-pyridin-3-ylmethyl)-6-(tetrahydro-pyran-4-ylmethoxy)-1,3-dihydro-imidazo[4,5-c]pyridin-2-one has been found to crystallise in two, anhydrous polymorphs designated Form A and Form B. These two forms can be distinguished by their Powder X-Ray Diffraction patterns.

### Characterisation

### (a) Powder X-Ray Diffraction (PXRD)

PXRD patterns were determined using a Bruker-AXS Ltd. D4 powder X-ray diffractometer fitted with an automatic sample changer, a theta-theta goniometer, automatic beam divergence slit, and a PSD Vantec-1 detector, calibrated for peak 2-theta positions against a Corundum standard (NIST: SRM 1976 XRD). The sample was prepared for analysis by mounting on a low background silicon wafer specimen mount. The specimen was rotated whilst being irradiated with copper K-alphal X-rays (wavelength = 1.5406 Angstroms) with the X-ray tube operated at 40kV/30mA. The analyses were performed with the goniometer running in continuous mode set for a 0.2 second count per 0.018° step over a 2-theta range of 2° to 55°. Peaks were selected manually using Bruker-AXS Ltd. evaluation software.

As will be appreciated by the skilled person, the relative intensities of the various peaks given below may vary due to a number of factors such as for example orientation effects of crystals in the X-ray beam or the purity of the material being analysed or the degree of crystallinity of the sample. The peak positions may also shift for variations in sample height but the peak positions will remain substantially as defined.

The skilled person will also appreciate that measurements using a different wavelength will result in different shifts according to the Bragg equation - nλ = 2d sin θ. Such further PXRD patterns generated by use of alternative wavelengths are considered to be alternative representations of the PXRD patterns of the crystalline materials of the present invention and as such are within the scope of the present invention

The PXRD pattern for Form A is shown in Figure 1. The main 2-theta peak positions and relative intensities are listed in Table 1. Form A displays characteristic diffraction peaks at 7.6, 13.3, 15.3 and 25.0 degrees 2-theta (± 0.1 degrees).

The PXRD pattern for Form B is shown in Figure 2. The main 2-theta peak positions and relative intensities are listed in Table 2. Form B displays characteristic diffraction peaks at 7.3, 17.9, 20.3, 24.0 and 24.3 degrees 2-theta (± 0.1 degrees).

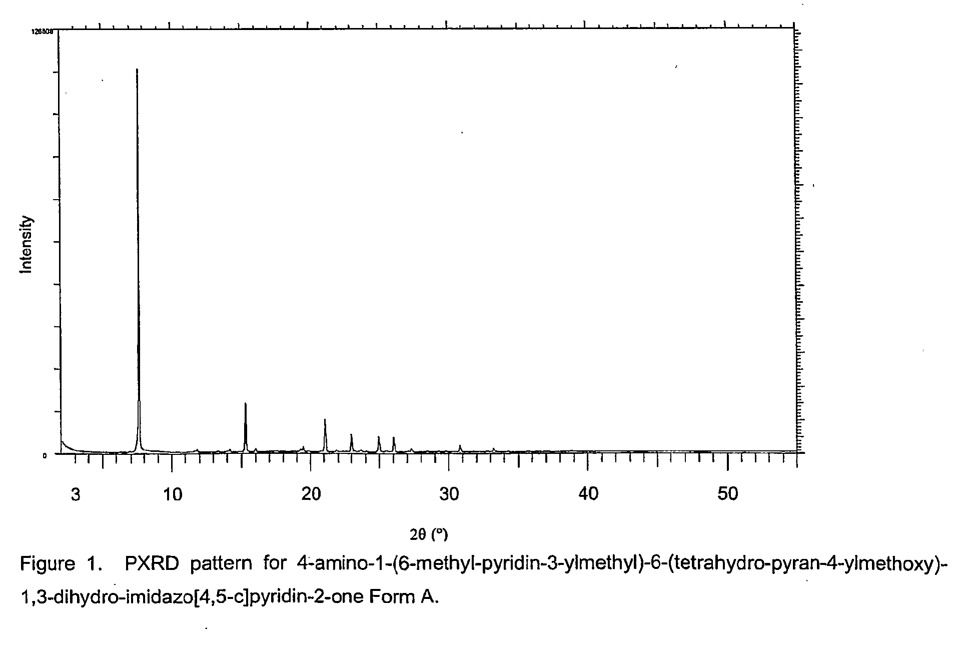

**Table 1. 2-theta peak positions (± 0.1 degrees) and relative intensities for the diffraction peaks observed for the 4-amino-1-(6-methyl-pyridin-3-ylmethyl)-6-(tetrahydro-pyran-4-ylmethoxy)-1,3-dihydro-imidazo[4,5-c]pyridin-2-one Form A PXRD pattern.**

| Angle 2-Theta (°) | Intensity (%) | Angle 2-Theta (°) | Intensity (%) | Angle 2-Theta (°) | Intensity (%) |
|---|---|---|---|---|---|
| 7.6 | 100 | 19.4 | 1.7 | 26.0 | 4.1 |
| 8.3 | 0.9 | 19.8 | 0.6 | 27.3 | 1.2 |
| 9.0 | 0.7 | 21.1 | 8.8 | 28.5 | 0.6 |
| 11.6 | 0.6 | 21.9 | 0.8 | 30.8 | 2.1 |
| 11.8 | 1.2 | 22.4 | 0.6 | 33.2 | 1.4 |
| 13.3 | 0.7 | 22.6 | 0.5 | 38.8 | 0.7 |
| 14.1 | 1.2 | 23.0 | 4.7 | | |
| 15.3 | 12.9 | 23.3 | 0.6 | | |
| 16.0 | 1.2 | 23.7 | 0.9 | | |
| 17.6 | 0.6 | 25.0 | 4.3 | | |
| 19.2 | 1.0 | 25.5 | 0.7 | | |

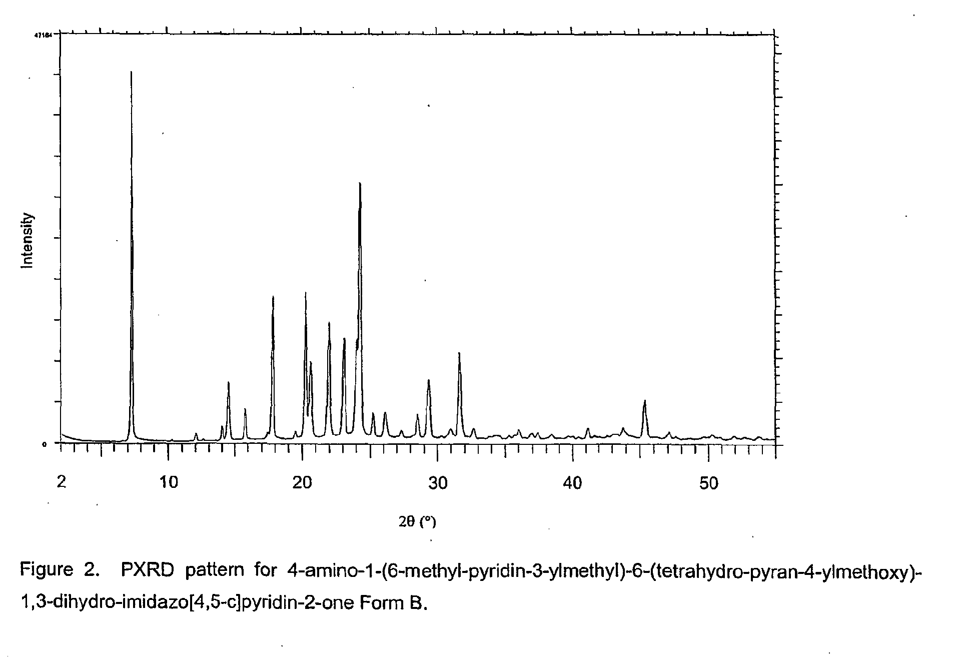

**Table 2. 2-theta peak positions (+ 0.1 degrees) and relative intensities for the diffraction peaks observed for the 4-amino-1-(6-methy)-pyridin-3-ylmethy)-6-(tetrahydro-pyran-4-ylmethoxy)-1,3-dihydro-imidazo[4,5-c]pyridin-2-one Form B PXRD.**

| Angle 2-Theta (°) | Intensity (%) | Angle 2-Theta (°) | Intensity (%) | Angle 2-Theta (°) | Intensity (%) |
|---|---|---|---|---|---|
| 7.3 | 100 | 22.0 | 32.7 | 36.1 | 3.6 |
| 10.2 | 1.0 | 23.1 | 28.2 | 37.0 | 2.8 |
| 12.0 | 2.5 | 24.0 | 27.9 | 37.5 | 2.9 |
| 14.0 | 4.3 | 24.3 | 70.1 | 38.5 | 2.5 |
| 14.5 | 16.3 | 25.2 | 8.2 | 41.2 | 4.1 |
| 15.8 | 9.0 | 26.2 | 8.5 | 43.1 | 2.5 |
| 17.5 | 3.0 | 28.6 | 7.8 | 43.8 | 4.3 |
| 17.8 | 39.5 | 29.4 | 17.2 | 45.4 | 11.6 |
| 19.5 | 3.2 | 31.0 | 4.0 | 47.2 | 3.1 |
| 20.3 | 40.5 | 31.7 | 24.1 | 50.4 | 2.5 |
| 20.7 | 21.9 | 32.7 | 4.0 | | |

### Biological data

The ability of the compounds of the invention to agonise TLR7 activity is demonstrated by a PBUHCV replicon bioassay as detailed below, in which the following abbreviations are used:
EMCV: Encephalomyocarditis virus
IRES: Internal ribosmomal entry site
Huh: Huh-7 human hepatoma cell line 7 (parental cells used to generate HCV replicon cell lines)
luc : luciferase
ubi : ubiquitin
neo: neomycin
ET: glutamic acid, threonine (cell culture adaptive mutations in the replicon used in the assay)
RPMI-FCS: Roswell Park Memorial Institute (cell culture medium for PBL) - Foetal Calf Serum
PBL: peripheral blood lymphocytes

PBL contain as a subpopulation piasmacytoid dendritic cells which are the natural interferon producing cells during an infection and as such are an excellent model in which to profile interferon inducers. As an extremly sensitive antiviral bioassay, supernatant taken from PBL is assayed for antiviral activity in the HCV replicon system. Antiviral EC50 values are defined as the concentration of a test compound applied to PBL that results in a 50% reduction of HCV replicon levels on transfer of a defined amount of PBL culture medium to a HCV replicon containing cell line. Although HCV replicon containing cells are fully responsive to PBL conditioned medium they do not respond directly to known TLR agonists such as Resiquimod and Imiquimod.

The HCV replicon (Huh-5-2[I389luc-ubi-neo-NS3-3'/ET]) is an in vitro model of HCV replication in which the luciferase reporter is incorporated into HCV sequences and stably maintained in the human hepatoma cell line Huh-7. The firefly luciferase reporter is expressed as a luciferase-ubiquitin-neomycin phosphotransferase fusion protein which is cleaved by host proteases to release luciferase. The replicon also contains an internal EMCV IRES, which drives translation of HCV NS3-5B polyprotein, which harbour cell culture adapted mutations to permit high cloning efficiency. The luciferase output has been shown to be directly proportional to the level of HCV RNA present in the host cell. Firefly luciferase activity is detected using a Bright-Glo™ Luciferase Assay System manufactured by Promega.

Typically, 1 - 3 mg of test compound is dissolved in 100%(v/v) DMSO to a final concentration of usually 1, 4 or 10 mM, or higher depending on the starting concentration required in the assay. An initial 3 fold serial dilution series of compounds in 100% DMSO is prepared from stocks. The dilution series is then further diluted 100 fold with complete RPMI - FCS. The final concentration of DMSO in the assay is thus 0.1% and that of the test compound is 1/1000 in the 100% DMSO dilution series.

PBL are prepared seeded at 5x105 /well/90µl into the previously prepared compound containing assay plates (96 well clear bottomed TC grade) and incubated for 24h.

LucUbiNeo HCV replicon cells are seeded at 10⁴ /well/90µl. These are incubated for 24h. After 24h, 10µl of medium is transferred from the PBL assay plates to the HCV replicon plates and incubated for a further 48h.

| Example No | 1 | 2 | 3 | 4 | 5 | 6 | 7 | 8 |
|---|---|---|---|---|---|---|---|---|
| EC₅₀(nM) | 105 | 128 | 1140 | 1470 | 1160 | 1940 | 54 | 112 |
| | 111 | 23 | 107 | 1250 | 475 | >4000 | 60 | |
| (n=no of experiment) | 104 | 187 | 1160 | 2070 | 1430 | >4000 | 91 | 87 |
| | 108 | 863 | 399 | 3270 | 1040 | 1470 | 40 | (n=2) |
| | 55 | 1220 | 414 | 1670 | 583 | >4000 | 1240 | |
| | 98 | 1320 | 489 | 2100 | 1010 | 1250 | 913 | |
| | 106 | (n=6) | (n=6) | (n=6) | 737 | 1580 | 3420 | |
| | 113 | | | | 698 | >4000 | | |
| | 113 | | | | 1240 | >4000 | (n=7) | |
| | 90 | | | | 1160 | 1000 | | |
| | 59 | | | | 3700 | 1450 | | |
| | 282 329 | | | | (n=11 ) | (n=11 ) | | |
| | 329 | | | | | | | |
| | 185 | | | | | | | |
| | 158 | | | | | | | |
| | 87 | | | | | | | |
| | 112 | | | | | | | |
| | 119 | | | | | | | |
| | | | | | | | | |
| | (n = 18) | | | | | | | |

It is desirable that the compounds of the invention have selectivity for TLR7 over other known TLRs. It is also desirable that the compounds of the invention have selectivity for TLR7 over cellular kinases and/or purinergic receptors, such as adenosine or phosphodiesterase receptors.

The compound of Example 1 was tested and found to be selective for TLR7 over the known TLRs 2-5 and 7-9.

In addition, Example 1 was tested and found to be selective for TLR7 over cellular kinases, phosphodiesterase receptors and adenosine receptors.

## Claims

1. A compound of formula (I) 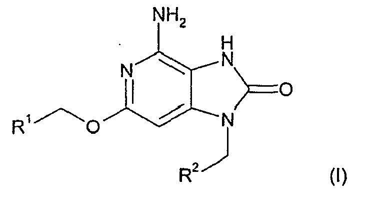 or a pharmaceutically acceptable salt or solvate thereof, wherein:
R¹ is 3- to 8-membered saturated heterocyclic group wherein one ring member is -O-; and
R² is phenyl or pyridinyl, each optionally substituted by C₁-C₆alkyl.

2. A compound according to claim 1 wherein R¹ is tetrahydropyranyl or tetrahydrofuranyl.

3. A compound according to claim 1 or 2 wherein R¹ is tetrahydropyranyl.

4. A compound according to any preceding claim wherein the R² is pyridinyl, optionally substituted by C₁-C₄alkyl.

5. A compound according to any preceding claim wherein R² is pyridinyl, optionally substituted by methyl.

6. A compound according to any of claims 1 to 4 wherein R² is pyridin-3-yl, optionally substituted by C₁-C₄alkyl

7. A compound according to claim 5 or 6 wherein R² is pyridin-3-yl optionally substituted by methyl.

8. A compound of formula (I) according to claim 1 which is selected from:
4-Amino-1-(6-methyl-pyridin-3-ylmethyl)-6-(tetrahydro-pyran-4-ylmethoxy)-1,3-dihydro-imidazo[4,5-c]pyridin-2-one;
4-Amino-1-(6-methyl-pyridin-3-ylmethyl)-6-(tetrahydro-furan-3-R/S-ylmethoxy)-1,3-dihydro-imidazo[4,5-c]pyridin-2-one;
4-Amino-1-(6-methyl-pyridin-3-ylmethyl)-6-(tetrahydro-furan-3-S-ylmethoxy)-1,3-dihydro-imidazo[4,5-c]pyridin-2-one;
4-Amino-1-(6-methyl-pyridin-3-ylmethyl)-6-(tetrahydro-furan-3-R-ylmethoxy)-1,3-dihydro-imidazo[4,5-c]pyridin-2-one;
4-Amino-1-(6-methyl-pyridin-3-ylmethyl)-6-(tetrahydro-furan-2-R/S-ylmethoxy)-1,3-dihydro-imidazo[4,5-c]pyridin-2-one;
4-Amino-1-(6-methyl-pyridin-3-ylmethyl-6-(tetrahydro-furan-2-R-ylmethoxy)-1,3-dihydro-imidazo[4,5-c]pyridin-2-one;
4-Amino-1-(6-methyl-pyridin-3-ylmethyl)-6-(tetrahydro-furan-2-S-ylmethoxy)-1,3-dihydro-imidazo[4,5-c]pyridin-2-one;
or a pharmaceutically acceptable salt or solvate thereof.

9. A pharmaceutical composition comprising a compound of formula (I) or a pharmaceutically acceptable salt or solvate thereof, as defined in any preceding claim, together with one or more pharmaceutically acceptable exciplents.

10. A pharmaceutical composition according to claim 9 including one or more additional therapeutic agents.

11. A compound of formula (I) or a pharmaceutically acceptable salt or solvate thereof, as defined in any of claims 1 to 8, for use as a medicament.

12. A compound of formula (I) or a pharmaceutically acceptable salt or solvate thereof, as defined in any of claims 1 to 8, for use in the treatment of a disorder for which a TLR7 agonist is indicated.

13. A compound of claim 12, wherein the disorder for which a TLR7 agonist is indicated is an infection caused by a virus selected from adenovirus, herpesvirus, poxvirus, picornavirus, orthomyxovirus, paramyxovirus, coronavirus, papovavirus, papillomavirus, hepadnavirus, flavivirus, retrovirus and filovirus.

14. A compound of claim 13 wherein the disorder for which a TLR7 agonist is indicated is hepatitis C.

15. Use of a compound of formula (I) or a pharmaceutically acceptable salt or solvate thereof, as defined in as claimed in any one of claims 1 to 8, for the preparation of a medicament for the treatment of a disorder for which a TLR7 agonist is indicated.

## Patentansprüche

1. Verbindung der Formel (I) 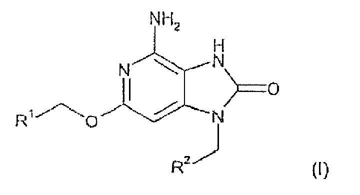 oder ein pharmazeutisch verträgliches Salz oder Solvat davon, worin:
R¹ eine 3- bis 8-gliedrige gesättigte heterocyclische Gruppe ist, in der ein Ringglied -O- ist, und
R² Phenyl oder Pyridinyl, jedes gegebenenfalls mit C₁-C₆-Alkyl substituiert, ist.

2. Verbindung gemäß Anspruch 1, wobei R¹ Tetrahydropyranyl oder Tetrahydrofuranyl ist.

3. Verbindung gemäß Anspruch 1 oder 2, wobei R¹ Tetrahydropyranyl ist.

4. Verbindung gemäß einem Vorangehenden Anspruch, wobei R² Pyridinyl, gegebenenfalls mit C₁-C₄-Alkyl substituiert, ist.

5. Verbindung gemäß einem vorangehenden Anspruch, wobei R² Pyridinyl, gegebenenfalls mit Methyl substituiert, ist.

6. Verbindung gemäß einem der Ansprüche 1 bis 4, wobei R² Pyridin-3-yl, gegebenenfalls mit C₁-C₄-Alkyl substituiert, ist.

7. Verbindung gemäß Anspruch 5 oder 6, wobei R² Pyridin-3-yl, gegebenenfalls substituiert mit Methyl, ist.

8. Verbindung gemäß Formel (I) gemäß Anspruch 1, die ausgewählt ist aus:
4-Amino-1-(6-methyl-pyridin-3-ylmethyl)-6-(tetrahydropyran-4-ylmethoxy)-1,3-dihydro-imidazo[4,5-c]pyridin-2-on;
4-Amino-1-(6-methyl-pyridin-3-ylmethyl)-6-(tetrahydrofuran-3-R/S-ylmethoxy)-1,3-dihydro-imidazo-[4,5-c]pyridin-2-on;
4-Amino-1-(6-methyl-pyridin-3-ylmethyl)-6-(tetrahydrofuran-3-S-ylmethoxy)-1,3-dihydro-imidazo[4,5-c]pyridin-2-on;
4-Amino-1-(6-methyl-pyridin-3-ylmethyl)-6-(tetrahydrofuran-3-R-ylmethoxy)-1,3-dihydro-imidazo[4,5-c]pyridin-2-on;
4-Amino-1-(6-methyl-pyridin-3-ylmethyl)-6-(tetrahydrofuran-2-R/S-ylmethoxy)-1,3-dihydro-imidazo[4,5-c]pyridin-2-on;
4-Amino-1-(6-methyl-pyridin-3-ylmethyl)-6-(tetrahydrofuran-2-R-ylmethoxy)-1,3-dihydro-imidazo[4,5-c]pyridin-2-on;
4-Amino-1-(6-methyl-pyridin-3-ylmethyl)-6-(tetrahydrofuran-2-S-ylmethoxy)-1,3-dihydro-imidazo[4,5-c]pyridin-2-on;
oder ein pharmazeutisch verträgliches Salz oder Solvat davon.

9. Pharmazeutische Zusammensetzung, umfassend eine Verbindung der Formel (I) oder ein pharmazeutisch verträgliches Salz oder Solvat davon, wie in einem vorangehenden Anspruch definiert, zusammen mit einem oder mehreren pharmazeutisch verträglichen Exzipiens/pharmazeutisch verträglichen Exzipienzien.

10. Pharmazeutische Zusammensetzung gemäß Anspruch 9, die ein oder mehrere zusätzliche therapeutische Mittel umfasst.

11. Verbindung der Formel (I) oder ein pharmazeutisch verträgliches Salz oder Solvat davon, wie in einem der Ansprüche 1 bis 8 definiert, zur Verwendung als Medikament.

12. Verbindung der Formel (I) oder ein pharmazeutisch verträgliches Salz oder Solvat davon, wie in einem der Ansprüche 1 bis 8 definiert, zur Verwendung bei der Behandlung einer Störung, für welche ein TLR7-Agonist indiziert ist.

13. Verbindung gemäß Anspruch 12, wobei die Störung, für welche ein TLR7-Agonist indiziert ist, eine Infektion ist, die durch ein Virus, ausgewählt aus Adenovirus, Herpesvirus, Poxvirus, Picornavirus, Orthomyxovirus, Paramyxovirus, Coronavirus, Papovavirus, Papillomavirus, Hepadnavirus, Flavivirus, Retrovirus und Filovirus, verursacht wird.

14. Verbindung gemäß Anspruch 13, wobei die Störung, für welche ein TLR7-Agonist indiziert ist, Hepatitis C ist.

15. Verwendung einer Verbindung der Formel (I) oder eines pharmazeutisch verträglichen Salzes oder Solvats davon, wie in einem der Ansprüche 1 bis 8 definiert, für die Herstellung eines Medikaments für die Behandlung einer Störung, für welche ein TLR7-Agonist indiziert ist.

## Revendications

1. Composé de formule (I) 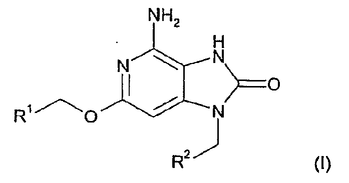 ou un de ses sels ou produits de solvatation pharmaceutiquement acceptables, formule dans laquelle :
R¹ représente un groupe hétérocyclique saturé tri- à octogonal dans lequel un membre du noyau est un groupe -O- ; et
R² représente un groupe phényle ou pyridinyle, chacun facultativement substitué avec un substituant alkyle en C₁ à C₆.

2. Composé suivant la revendication 1, dans lequel R¹ représente un groupe tétrahydropyrannyle ou tétrahydrofurannyle.

3. Composé suivant la revendication 1 ou 2, dans lequel R¹ représente un groupe tétrahydropyrannyle.

4. Composé suivant l'une quelconque des revendications précédentes, dans lequel R² représente un groupe pyridinyle, facultativement substitué avec un substituant alkyle en C₁ à C₄.

5. Composé suivant l'une quelconque des revendications précédentes, dans lequel R² représente un groupe pyridinyle, facultativement substitué avec un substituant méthyle.

6. Composé suivant l'une quelconque des revendications 1 à 4, dans lequel R² représente un groupe pyridine-3-yle, facultativement substitué avec un substituant alkyle en C₁ à C₄.

7. Composé suivant la revendication 5 ou 6, dans lequel R² représente un groupe pyridine-3-yle, facultativement substitué avec un substituant méthyle.

8. Composé de formule (I) suivant la revendication 1, qui est choisi entre :
la 4-amino-1-(6-méthyl-pyridine-3-ylméthyl)-6-(tétrahydropyranne-4-ylméthoxy)-1,3-dihydro-imidazo[4,5-c]pyridine-2-one ;
la 4-amino-1-(6-méthyl-pyridine-3-ylméthyl)-6-(tétrahydrofuranne-3-R/S-ylméthoxy)-1,3-dihydro-imidazo[4,5-c]pyridine-2-one ;
la 4-amino-1-(6-méthyl-pyridine-3-ylméthyl)-6-(tétrahydrofuranne-3-S-ylméthoxy)-1,3-dihydro-imidazo[4,5-c]pyridine-2-one ;
la 4-amino-1-(6-méthyl-pyridine-3-ylméthyl)-6-(tétrahydrofuranne-3-R-ylméthoxy)-1,3-dihydro-imidazo[4,5-c]pyridine-2-one ;
la 4-amino-1-(6-méthyl-pyridine-3-ylméthyl)-6-(tétrahydrofuranne-2-R/S-ylméthoxy)-1,3-dihydro-imidazo[4,5-c]pyridine-2-one ;
la 4-amino-1-(6-méthyl-pyridine-3-ylméthyl)-6-(tétrahydrofuranne-2-R-ylméthoxy)-1,3-dihydro-imidazo[4,5-c]pyridine-2-one ;
la 4-amino-1-(6-méthyl-pyridine-3-ylméthyl)-6-(tétrahydrofuranne-2-S-ylméthoxy)-1,3-dihydro-imidazo[4,5-c]pyridine-2-one ;
ou un de ses sels ou produits de solvatation pharmaceutiquement acceptables.

9. Composition pharmaceutique comprenant un composé de formule (I) ou un de ses sels ou produits de solvatation pharmaceutiquement acceptables tel que défini dans l'une quelconque des revendications précédentes, conjointement avec un ou plusieurs excipients pharmaceutiquement acceptables.

10. Composition pharmaceutique suivant la revendication 9, comprenant un ou plusieurs agents thérapeutiques supplémentaires.

11. Composé de formule (I) ou un de ses sels ou produits de solvatation pharmaceutiquement acceptables, tel que défini dans l'une quelconque des revendications 1 à 8, destiné à être utilisé comme médicament.

12. Composé de formule (I) ou un de ses sels ou produits de solvatation pharmaceutiquement acceptables, tel que défini dans l'une quelconque des revendications 1 à 8, destiné à être utilisé dans le traitement d'un trouble pour lequel un agoniste de TLR7 est indiqué.

13. Composé suivant la revendication 12, dans lequel trouble pour lequel un agoniste de TLR7 est indiqué est une infection provoquée par un virus choisi entre un adénovirus, un herpesvirus, un poxvirus, un picornavirus, un orthomyxovirus, un paramyxovirus, un coronavirus, un papovavirus, un papillomavirus, un hépadnavirus, un flavivirus, un rétrovirus et un filovirus.

14. Composé suivant la revendication 13, dans lequel le trouble pour lequel un agoniste de TLR7 est indiqué est l'hépatite C.

15. Utilisation d'un composé de formule (I) ou d'un de ses sels ou produits de solvatation pharmaceutiquement acceptables, tel que défini dans l'une quelconque des revendications 1 à 8, pour la préparation d'un médicament destiné au traitement d'un trouble pour lequel un agoniste de TLR7 est indiqué.
